# FASCICULE DE BREVET EUROPEEN

(11) **EP 1 280 898 B1**
(45) Date de publication et mention de la délivrance du brevet: **09.02.2011**
(21) Numéro de dépôt: 01928006.4
(22) Date de dépôt: 19.04.2001
(51) Int. Cl.: C12N 15/12, G01N 33/68, A01K 67/027, C07K 14/465, C07K 16/18, A61K 39/395, A61K 31/7088, A61K 38/17, C12N 5/10, C12N 15/63, C12Q 1/68

(54) **CELLULES ES MODIFIEES ET GENE SPECIFIQUE DE CELLULES ES**
MODIFIZIERTE EMBRYONALE STAMMZELLEN UND EMBRYONALES STAMMZELLSPEZIFISCHES GEN
MODIFIED ES CELLS AND ES CELL-SPECIFIC GENE

(30) Priorité: 11.05.2000 FR 0006029
(43) Date de publication de la demande: 05.02.2003
(73) Titulaire: INSTITUT NATIONAL DE LA RECHERCHE AGRONOMIQUE, 75007 Paris (FR); CENTRE NATIONAL DE LA RECHERCHE SCIENTIFIQUE (CNRS), 75794 Paris Cedex 16 (FR); ECOLE NORMALE SUPERIEURE DE LYON, 69007 Lyon (FR)
(72) Inventeur: ACLOQUE, Hervé, F-69007 Lyon (FR); BIROT, Anne-Marie, F-69110 Sainte-Foy-les-Lyon (FR); RISSON, Valérie, F-69100 Villeurbanne (FR); PAIN, Bertrand, F-69003 Lyon (FR); SAMARUT, Jacques, F-69100 Villeurbanne (FR)
(74) Mandataire: Warcoin, Jacques
(86) Numéro de dépôt international: PCT/FR2001/001207
(87) Numéro de publication internationale: WO 2001/085938

(56) Documents cités:
- WO-A-00/12683
- DATABASE EMBL [en ligne] accession: AJ397754, 19 février 2000 (2000-02-19) BUERSTEDDE J M ET AL: "Gallus gallus EST clone 30n22r1" XP002164730 cité dans la demande
- DATABASE EMBL [en ligne] accession: AJ393785, 19 février 2000 (2000-02-19) BUERSTEDDE J M ET AL: "Gallus gallus EST clone 16n24r1" XP002164731 cité dans la demande
- ACLOQUE HERVE ET AL: "Identification of a new gene family specifically expressed in chicken embryonic stem cells and early embryo." MECHANISMS OF DEVELOPMENT, vol. 103, no. 1-2, mai 2001 (2001-05), pages 79-91, XP001015571 ISSN: 0925-4773 -& DATABASE EMBL [en ligne] accession: AF327879, 12 février 2001 (2001-02-12) ACLOQUE H ET AL: "Gallus gallus ENS-1 (ens-1) mRNA, complete cds." XP002173838
- PAIN B ET AL: "Long-term in vitro culture and characterisation of avian embryonic stem cells with multiple morphogenetic potentialities." DEVELOPMENT (CAMBRIDGE), vol. 122, no. 8, 1996, pages 2339-2348, XP002164729 ISSN: 0950-1991 cité dans la demande

### LISTE DE SÉQUENCES

<110> INSTITUT NATIONAL DE RECHERCHE AGRONOMIQUE - INRA CENTRE NATIONAL DE LA RECHERCHE SCIENTIFIQUE - CNRS ÉCOLE NORMAL SUPÉRIEURE DE LYON - ENS LYON
<120> CELLULES ES MODIFIÉES ET GÈNE SPÉCIFIQUE DE CELLULES ES.
<130> D18898
<150> FR 00 06029
   <151> 2000-05-11
<160> 15
<170> PatentIn Ver. 2.1
<210> 1
   <211> 4177
   <212> ADN
   <213> Poulet
<220>
   <221> CDS
   <222> (1409)..(2881)
<400> 1
   cgacagactt gaggggttct ctgccaactg atctctcacc gcaatgggta gacggatctc 60
   tacgtggaga ctgatctctc accacgacac gagcttcctg ccttccgatc ctcctctacg 120
   gaccgtttgc tgacggactt ccctgggcct gctacctgag acctgctgct tcctccctga 180
   cctgcatcct ctcgctgccc cagaccggcc tcgctgctcc tgcccttcgg cctcggaccg 240
   tcggaacatc gtgcaacggg actgctgccg gatcctggtg gtgactatcc ccgctttacg 300
   caattcttgc ctctttctat cttttctatc gctcgccttc ccttccccat caccccaatc 360
   cttaatagcg tccgtcctcc cctttcccca tctcccttat taacatttgt aataaactgg 420
   tcggaccaac atttgaaccg ctgtttctta atctcacgcc gggcatacat atttcaaaga 480
   acctcttctc cctcctataa attggagcga gacatttttt atggcgtagt cggcaggata 540
   cccgccgtga gagtgttgtc cttccagata atagtctgaa actttctgcg tgtacctcct 600
   ggagttgcaa gaagcgatac ttcttgataa cttagacgtg agcacctctc caggaagatc 660
   gcttcatact ctgaaacttt actatttatg tgtgtacctc tcgaggatgt atgaattttg 720
   tctaattgta tttatttaat acgtgtgtgc ctcctcggga agacctctct gcattttgtg 780
   aacccctctc tacgtgtgcg cctcttgggg aagcaagata cacgtttttt gacttaaaaa 840
   acttgtgtgc ctcccaagaa gttttctcac tttgctgaaa attgtttatg tatgcacctc 900
   tcgaggacgt atgaatcttg tctaattgca tttaatacgt gtgtgcctcc tcgggaagac 960
   ctctctgcat tttgtgactt aaggatcttg caacttaagt gtgaaatttg aacctctttc 1020
   gtgcgtgcct cttggggaag tgaggaagtg atacacgttt tttgatttaa aaaacgtgtg 1080
   cgcttctcca agaagtttat tcactttgtt aatcctagna aagtgttgtt ttagcttaaa 1140
   attaactgtg ggttttgaaa ccgaagtgtg ccttgctttg gtgtggtgtt tgcagttttt 1200
   tgtgtggctt cgcagggaag ttaggagcga ttttaagttg gtttagtctc tttgcccttg 1260
   tgctttcctc aacaaaggga ggcgcaatcg gaacatttac atttctttag ttgtggtgtg 1320
   cctccgtggg agaggcgata aggagttatt tgtacttttg aataggagta cctcctctct 1380
   cagtctatat ctttctgtgt atttggga atg agc aac agt atg gcc agt atg    1432
                                   Met Ser Asn Ser Met Ala Ser Met
                                   1               5
   aaa agt gaa gat gta tta ttt gat ctt tta gaa aag cat ggt gct cgg    1480
   Lys Ser Glu Asp Val Leu Phe Asp Leu Leu Glu Lys His Gly Ala Arg
       10                  15                  20
   cct tct gta tca ggg gtg gat tgg gca cga cag aac tgg tat aat ttg    1528
   Pro Ser Val Ser Gly Val Asp Trp Ala Arg Gln Asn Trp Tyr Asn Leu
   25                  30                  35                  40
   caa agt gtt tca gac cgt att cgt gtt tta caa aat gag gct cgt act    1576
   Gln Ser Val Ser Asp Arg Ile Arg Val Leu Gln Asn Glu Ala Arg Thr
                   45                  50                  55
   cgg gcc gga aaa ggg aaa tct ttt att tgt gca gta ctc ggt gct gct    1624
   Arg Ala Gly Lys Gly Lys Ser Phe Ile Cys Ala Val Leu Gly Ala Ala
               60                  65                  70
   tta aaa gca gct gtg gag ttc cga gag gaa aag aac tct acg gaa acc    1672
   Leu Lys Ala Ala Val Glu Phe Arg Glu Glu Lys Asn Ser Thr Glu Thr
           75                  80                  85
   cag agt att caa gca tta cag gaa tcg gtt aaa gtg acg caa gaa ttg    1720
   Gln Ser Ile Gln Ala Leu Gln Glu Ser Val Lys Val Thr Gln Glu Leu
       90                  95                  100
   gta aaa tct ctg caa agc caa ata agg agt ctt gag gat caa tta gaa    1768
   Val Lys Ser Leu Gln Ser Gln Ile Arg Ser Leu Glu Asp Gln Leu Glu
   105                 110                 115                 120
   aga gaa aaa cac aat tcg gtt ctg ttg caa aca gct ttt aag gag ctg    1816
   Arg Glu Lys His Asn Ser Val Leu Leu Gln Thr Ala Phe Lys Glu Leu
                   125                 130                 135
   ata acg tgt aag gac acc ggt gac act gtt atc cac agt gca cct caa    1864
   Ile Thr Cys Lys Asp Thr Gly Asp Thr Val Ile His Ser Ala Pro Gln
               140                 145                 150
   gaa aaa gtt tat cct caa ggg aaa tta caa gag gtg aag gaa agg cta    1912
   Glu Lys Val Tyr Pro Gln Gly Lys Leu Gln Glu Val Lys Glu Arg Leu
           155                 160                 165
   gat aaa tta gag gcc tct cca gcc cac att cgt cct ttg ata aaa act    1960
   Asp Lys Leu Glu Ala Ser Pro Ala His Ile Arg Pro Leu Ile Lys Thr
       170                 175                 180
   gaa tat act ttc gat aac agt gag aat cta gat cct caa atg aat gtt    2008
   Glu Tyr Thr Phe Asp Asn Ser Glu Asn Leu Asp Pro Gln Met Asn Val
   185                 190                 195                 200
   aag gaa att ccc ttt tcg gcc act gaa ctg gcc aaa ctg aaa aag gat      2056
   Lys Glu Ile Pro Phe Ser Ala Thr Glu Leu Ala Lys Leu Lys Lys Asp
                   205                 210                 215
   ttc agt cgc tcc cca aag gag tct gaa aca gag tac gtc tgg aga gtt      2104
   Phe Ser Arg Ser Pro Lys Glu Ser Glu Thr Glu Tyr Val Trp Arg Val
               220                 225                 230
   agt ctc act ggc gga gac cag atc cta cta aca gag aaa gaa gct gaa      2152
   Ser Leu Thr Gly Gly Asp Gln Ile Leu Leu Thr Glu Lys Glu Ala Glu
           235                 240                 245
   ggt tac tgg gga cca gga gta ttt tta acc act ggc aat aat cgt gct      2200
   Gly Tyr Trp Gly Pro Gly Val Phe Leu Thr Thr Gly Asn Asn Arg Ala
       250                 255                 260
   ccc tgg tcc tta aca cag agg gct gcc tat tgg gca ggg ggt ctc aac      2248
   Pro Trp Ser Leu Thr Gln Arg Ala Ala Tyr Trp Ala Gly Gly Leu Asn
   265                 270                 275                 280
   cct tta gaa agg ggg gac cct ctt gct att act gga act atc gac cag      2296
   Pro Leu Glu Arg Gly Asp Pro Leu Ala Ile Thr Gly Thr Ile Asp Gln
                   285                 290                 295
   tta gtg gag aat gtt cag aaa gct gct tgt ctc caa atg atg tat gat      2344
   Leu Val Glu Asn Val Gln Lys Ala Ala Cys Leu Gln Met Met Tyr Asp
               300                 305                 310
   aga aag ttg cag cca cat aat gaa tca ccc atg atg tta cct gtt aat      2392
   Arg Lys Leu Gln Pro His Asn Glu Ser Pro Met Met Leu Pro Val Asn
           315                 320                 325
   ccg gag aga ctg aca cct cta atc agg gga ctt cct gaa tcg tta aaa      2440
   Pro Glu Arg Leu Thr Pro Leu Ile Arg Gly Leu Pro Glu Ser Leu Lys
       330                 335                 340
   cct ata ggt ata caa ctc caa gga aag ata caa gcc atg tct cag gga      2488
   Pro Ile Gly Ile Gln Leu Gln Gly Lys Ile Gln Ala Met Ser Gln Gly
   345                 350                 355                 360
   gag aga acc tgg gca gcg ttg gag gga tct gta gcc cct aac cac cag      2536
   Glu Arg Thr Trp Ala Ala Leu Glu Gly Ser Val Ala Pro Asn His Gln
                   365                 370                 375
   tca gga ccc aaa gtg tgg act tgg gga gag gtt gcc caa gaa tta att      2584
   Ser Gly Pro Lys Val Trp Thr Trp Gly Glu Val Ala Gln Glu Leu Ile
               380                 385                 390
   aac tat gga aga aaa tat ggg ccg gtg gtt tct acc tgc agt aaa ttt      2632
   Asn Tyr Gly Arg Lys Tyr Gly Pro Val Val Ser Thr Cys Ser Lys Phe
           395                 400                 405
   gag cca aga gga gta agg ctt gca gta gcc agc ctt gcc tcc agg cct      2680
   Glu Pro Arg Gly Val Arg Leu Ala Val Ala Ser Leu Ala Ser Arg Pro
       410                 415                 420
   cct agc cca aga ctt att gga acc aaa aag gtt tca tcc cca gta aaa      2728
   Pro Ser Pro Arg Leu Ile Gly Thr Lys Lys Val Ser Ser Pro Val Lys
   425                 430                 435                 440
   acg ggg aca cga tgc att gat cat aaa cgc aat gga ctt tgg acn ctg    2776
   Thr Gly Thr Arg Cys Ile Asp His Lys Arg Asn Gly Leu Trp Xaa Leu
                   445                 450                 455
   ggc tgg aca aag ggt att cca cga gat ttg atg aat gga tta ccc aca    2824
   Gly Trp Thr Lys Gly Ile Pro Arg Asp Leu Met Asn Gly Leu Pro Thr
               460                 465                 470
   gtc aga tta gag aaa tta gtt aac tgc tgg cca gaa caa aag ctc aag    2872
   Val Arg Leu Glu Lys Leu Val Asn Cys Trp Pro Glu Gln Lys Leu Lys
           475                 480                 485
   ggg agc tga tgccttcgcc cccccctccc aggtgagcgg gaggtgggtg            2921
   Gly Ser
       490
   ggggggtgaa gggtggatgt ttattaggaa gctcacgact aaaggaaaca atctgttaat 2981
   tgtttattta ttattagtgg ttattgtcaa atgtacggtt gtctcttttc tctcttctat 3041
   tcattatgta atattcatgt taccactcct gaagaatcac ggggtggtgt ctatggcaag 3101
   ttgcattgtg tactgttgca actcttatgt ttgtatgatt ccatgtttta tacaagatgt 3161
   tgtatcccct atttactttg taaccaaacc tgaaaaatgt ttgtaatgat tgtatgaaac 3221
   atttgattcc acaacccctc cctcctttac ccttgtgctt gctatcttct ctcaccacca 3281
   tggatgccca gtgtccaatt tttaagcaac ctttgagtca cggggtggtg taagagacta 3341
   ttcttttata tcattgactc aaagtttgct gaggaacaag tccaggcaag tcctgggcaa 3401
   aggcagagaa atcttttgtc ttgaggacac tgatggacag gtcctggcta aggattgtga 3461
   aatcctttaa ggagcacaga tggacaaggc caggggcatc gagagagaga taagctgccg 3521
   ctaatggccg ggaaacggtc tttttgtgtg gacttatctc aaggaaaatg gccatctcag 3581
   gaggtatgca caggactctt gctcaagccc ccaggaatgt cacgtaggca gcagaaaatg 3641
   gaggataaaa gaggtccaat aaccacaacg gtggaagctg atccttcacc acaaccacgg 3701
   caacgggaga ngcttatctc tcaccacgac agacttgagg ggttctctgc caactgatct 3761
   ctcaccgcaa tgggtagacg gatctctacg tggagactga tctctcacca cgacacgagc 3821
   ttcctgcctt ccgatcctcc tctacggacc gtttgctgat ggacttccct gggcctgcta 3881
   cctgagacct gctgcttcct ccctgacctg catcctctcg ctgccccaga ccggcctcgc 3941
   tgctcctgcc cttccgtcgg aacatcgtgc aacgggactg ctgccggatc ctggtggtga 4001
   ctatccccgc ttaacgcaat tctngcctct ttctatcttt tntatcgctc gccttccctt 4061
   ccccatcacc ccaatcctta atagcgtccg tcctcccctt tccccatctc ccttattaac 4121
   atttgtaata aactggtcgg accaacaaaa aaaaaaaaaa aaaaaaaaaa aaaaaa     4177
<210> 2
   <211> 490
   <212> PRT
   <213> Poulet
<400> 2
   Met Ser Asn Ser Met Ala Ser Met Lys Ser Glu Asp Val Leu Phe Asp
    1               5                  10                  15
   Leu Leu Glu Lys His Gly Ala Arg Pro Ser Val Ser Gly Val Asp Trp
               20                  25                  30
   Ala Arg Gln Asn Trp Tyr Asn Leu Gln Ser Val Ser Asp Arg Ile Arg
           35                  40                  45
   Val Leu Gln Asn Glu Ala Arg Thr Arg Ala Gly Lys Gly Lys Ser Phe
       50                  55                  60
   Ile Cys Ala Val Leu Gly Ala Ala Leu Lys Ala Ala Val Glu Phe Arg
   65                  70                  75                  80
   Glu Glu Lys Asn Ser Thr Glu Thr Gln Ser Ile Gln Ala Leu Gln Glu
                   85                  90                  95
   Ser Val Lys Val Thr Gln Glu Leu Val Lys Ser Leu Gln Ser Gln Ile
               100                 105                 110
   Arg Ser Leu Glu Asp Gln Leu Glu Arg Glu Lys His Asn Ser Val Leu
           115                 120                 125
   Leu Gln Thr Ala Phe Lys Glu Leu Ile Thr Cys Lys Asp Thr Gly Asp
       130                 135                 140
   Thr Val Ile His Ser Ala Pro Gln Glu Lys Val Tyr Pro Gln Gly Lys
   145                 150                 155                 160
   Leu Gln Glu Val Lys Glu Arg Leu Asp Lys Leu Glu Ala Ser Pro Ala
                   165                 170                 175
   His Ile Arg Pro Leu Ile Lys Thr Glu Tyr Thr Phe Asp Asn Ser Glu
               180                 185                 190
   Asn Leu Asp Pro Gln Met Asn Val Lys Glu Ile Pro Phe Ser Ala Thr
           195                 200                 205
   Glu Leu Ala Lys Leu Lys Lys Asp Phe Ser Arg Ser Pro Lys Glu Ser
       210                 215                 220
   Glu Thr Glu Tyr Val Trp Arg Val Ser Leu Thr Gly Gly Asp Gln Ile
   225                 230                 235                 240
   Leu Leu Thr Glu Lys Glu Ala Glu Gly Tyr Trp Gly Pro Gly Val Phe
                   245                 250                 255
   Leu Thr Thr Gly Asn Asn Arg Ala Pro Trp Ser Leu Thr Gln Arg Ala
               260                 265                 270
   Ala Tyr Trp Ala Gly Gly Leu Asn Pro Leu Glu Arg Gly Asp Pro Leu
           275                 280                 285
   Ala Ile Thr Gly Thr Ile Asp Gln Leu Val Glu Asn Val Gln Lys Ala
       290                 295                 300
   Ala Cys Leu Gln Met Met Tyr Asp Arg Lys Leu Gln Pro His Asn Glu
   305                 310                 315                 320
   Ser Pro Met Met Leu Pro Val Asn Pro Glu Arg Leu Thr Pro Leu Ile
                   325                 330                 335
   Arg Gly Leu Pro Glu Ser Leu Lys Pro Ile Gly Ile Gln Leu Gln Gly
               340                 345                 350
   Lys Ile Gln Ala Met Ser Gln Gly Glu Arg Thr Trp Ala Ala Leu Glu
           355                 360                 365
   Gly Ser Val Ala Pro Asn His Gln Ser Gly Pro Lys Val Trp Thr Trp
       370                 375                 380
   Gly Glu Val Ala Gln Glu Leu Ile Asn Tyr Gly Arg Lys Tyr Gly Pro
   385                 390                 395                 400
   Val Val Ser Thr Cys Ser Lys Phe Glu Pro Arg Gly Val Arg Leu Ala
                   405                 410                 415
   Val Ala Ser Leu Ala Ser Arg Pro Pro Ser Pro Arg Leu Ile Gly Thr
               420                 425                 430
   Lys Lys Val Ser Ser Pro Val Lys Thr Gly Thr Arg Cys Ile Asp His
           435                 440                 445
   Lys Arg Asn Gly Leu Trp Xaa Leu Gly Trp Thr Lys Gly Ile Pro Arg
       450                 455                 460
   Asp Leu Met Asn Gly Leu Pro Thr Val Arg Leu Glu Lys Leu Val Asn
   465                 470                 475                 480
   Cys Trp Pro Glu Gln Lys Leu Lys Gly Ser
                   485                 490
<210> 3
   <211> 22
   <212> ADN
   <213> Escherichia coli
<220>
   <223> Gène LacZ.
<400> 3
   tggagtgacg gcagttatct gg 22
<210> 4
   <211> 22
   <212> ADN
   <213> Escherichia coli
<220>
   <223> Gène LacZ.
<400> 4
   ggcttcatcc accacataca gg 22
<210> 5
   <211> 25
   <212> ADN
   <213> Escherichia coli
<220>
   <223> Gène LacZ.
<400> 5
   ccgtgcatct gccagtttga gggga 25
<210> 6
   <211> 44
   <212> ADN
   <213> Séquence artificielle
<220>
   <223> Description de la séquence artificielle:Adaptateur amorce synthétique.
<400> 6
   ctaatacgac tcactatagg gctcgagcgg ccgcccgggc aggt 44
<210> 7
   <211> 27
   <212> ADN
   <213> Séquence artificielle
<220>
   <223> Description de la séquence artificielle: Amorce synthétique
<400> 7
   ccatcctaat acgactcact atagggc 27
<210> 8
   <211> 20
   <212> ADN
   <213> Escherichia coli
<220>
   <223> Gène LacZ.
<400> 8
   gggatccgcc atgtcacaga 20
<210> 9
   <211> 44
   <212> ADN
   <213> Poulet
<220>
   <223> Amorce.
<400> 9
   actatcgatt ctggaacctt cagaggtttt tttttttttt tttt 44
<210> 10
   <211> 21
   <212> ADN
   <213> Poulet
<220>
   <223> Amorce.
<400> 10
   gtcgtgcaac gggactgcct g 21
<210> 11
   <211> 25
   <212> ADN
   <213> Poulet
<220>
   <223> Amorce.
<400> 11
   ctatcgattc tggaaccttc agagg 25
<210> 12
   <2.11> 171
   <212> ADN
   <213> Poulet
<400> 12
<210> 13
   <211> 18
   <212> ADN
   <213> Poulet
<220>
   <223> Amorce.
<400> 13
   agaccggcct cactgctc 18
<210> 14
   <211> 21
   <212> ADN
   <213> Poulet
<220>
   <223> Amorce ens1 S1
<400> 14
   ggatctagat cctcaaatga a 21
<210> 15
   <211> 19
   <212> ADN
   <213> Poulet
<220>
   <223> Amorce ens1 AS1
<400> 15
   aattcttggg caacctctc 19

## Description

La présente invention concerne des cellules ES d'oiseau modifiées, exprimant spécifiquement un gène exogène lorsqu'elles possèdent un caractère pluripotent. L'invention concerne également un acide nucléique et un polypeptide exprimés spécifiquement dans les cellules d'oiseau pluripotentes, ainsi que des méthodes de détection du caractère pluripotent de cellules mettant en oeuvre ces acide nucléique et polypeptide.

Les cellules ES sont des cellules pluripotentes isolées d'embryon très précoce, qui sont capables de participer à la morphogenèse de tous les tissus incluant le tissu germinal, après leur transplantation dans des embryons hôtes. Ces cellules ont d'abord été isolées chez la souris où elles sont très largement utilisées pour la création d'animaux mutants portant des modifications très ciblées de leur génome. Des cellules ES ont été isolées et caractérisées chez les oiseaux (Pain et col. 1996). Ces cellules peuvent être utilisées pour modifier le patrimoine génétique du poulet (Etches et al. 1996, Pain et col. 1999). Un milieu de culture permettant de maintenir le caractère pluripotent de ces cellules d'oiseaux a fait l'objet de la demande de brevet WO 96/12793.

La difficulté rencontrée par tous ceux qui veulent isoler des cellules ES en culture, concerne l'identification rapide de ces cellules et de leur caractère pluripotent. Plusieurs marqueurs cellulaires ont été utilisés comme l'expression d'une activité phosphatase alcaline (Strickland et col. 1980), l'expression d'épitopes antigéniques (Kemler et col. 1981, Solter et Knowles 1978), l'expression de protéines spécifiques comme OCT-3 (Rosner et col. 1990), l'expression d'une activité télomérase (Prowse et Greider 1995). Les protéines OCT-3, REX-1, UTF-1 entre autres n'ont été identifiées jusqu'à présent que chez la souris. La vérification ultime du caractère pluripotent repose sur l'analyse des potentialités morphogénétiques de ces cellules après leur greffe dans des embryons hôtes, ce qui représente un test très lourd.

Une autre difficulté rencontrée avec la culture des cellules ES en culture comprend l'obtention de populations cellulaires d'un degré d'hétérogénéité faible et satisfaisant, et le problème du contrôle de la croissance en culture de cellules non-pluripotentes. En effet, un problème particulier associé à la présence continuelle de certains types cellulaires différenciés, c'est-à-dire que ces cellules sont capables d'éliminer les cellules ES de la culture en induisant leur différenciation ou leur mort cellulaire programmée.

La présente invention se propose de simplifier l'identification du caractère pluripotent de cellules d'oiseaux en culture, par la divulgation d'une séquence nucléique (gène *ens-1*) exprimée spécifiquement et sélectivement par les cellules pluripotentes.

Ainsi, la présente invention a pour objet un acide nucléique caractérisé en ce qu'il comprend une séquence nucléique choisie dans le groupe de séquences suivantes:
a) SEQ ID N° 1, ou le fragment nucléotidique 1409-2878 de SEQ IDN° 1 ;
b) le fragment nucléotidique 3111-3670 de SEQ ID N° 1 ;
c) une séquence nucléique présentant un pourcentage d'identité d'au moins 80 %, après alignement optimal avec une séquence définie en a) ou b), possédant un rôle dans le caractère de pluripotence des cellules ES et utilisable comme marqueur du caractère de pluripotence des cellules ES ;
d) la séquence complémentaire ou la séquence d'ARN issue de la séquence telle que définie en a), b) ou c).

De préférence, la base présente en 2773 de SEQ ID N° 1 est un « t », le codon correspondant codant alors pour une thréonine.

La séquence d'acides nucléiques selon l'invention définie en c) présente un pourcentage d'identité d'au moins 80 % après alignement optimal avec une séquence telle que définie en a) ou b) ci-dessus, de préférence 90 %, de façon la plus préférée 98 %. De préférence, la séquence définie en c), ou en d) est comparée à une des séquences définies en a).

Par acide nucléique, séquence nucléique ou d'acide nucléique, polynucléotide, oligonucléotide, séquence de polynucléotide, séquence nucléotidique, termes qui seront employés indifféremment dans la présente description, on entend désigner un enchaînement précis de nucléotides, modifiés ou non, permettant de définir un fragment ou une région d'un acide nucléique, comportant ou non des nucléotides non naturels, et pouvant correspondre aussi bien à un ADN double brin, un ADN simple brin que des produits de transcription desdits ADNs. Ainsi, les séquences nucléiques selon l'invention englobent également les PNA (Peptid Nucleic Acid), ou analogues.

Il doit être compris que la présente invention ne concerne pas les séquences nucléotidiques dans leur environnement chromosomique naturel, c'est-à-dire à l'état naturel. Il s'agit de séquences qui ont été isolées et/ou purifiées, c'est-à-dire qu'elles ont été prélevées directement ou indirectement, par exemple par copie, leur environnement ayant été au moins partiellement modifié. On entend ainsi également désigner les acides nucléiques obtenus par synthèse chimique.

Par « pourcentage d'identité » entre deux séquences d'acides nucléiques ou d'acides aminés au sens de la présente invention, on entend désigner un pourcentage de nucléotides ou de résidus d'acides aminés identiques entre les deux séquences à comparer, obtenu après le meilleur alignement, ce pourcentage étant purement statistique et les différences entre les deux séquences étant réparties au hasard et sur toute leur longueur. On entend désigner par "meilleur alignement" ou "alignement optimal", l'alignement pour lequel le pourcentage d'identité déterminé comme ci-après est le plus élevé. Les comparaisons de séquences entre deux séquences d'acides nucléiques ou d'acides aminés sont traditionnellement réalisées en comparant ces séquences après les avoir alignées de manière optimale, ladite comparaison étant réalisée par segment ou par « fenêtre de comparaison » pour identifier et comparer les régions locales de similarité de séquence. L'alignement optimal des séquences pour la comparaison peut être réalisé, outre manuellement, au moyen de l'algorithme d'homologie locale de Smith et Waterman (1981), au moyen de l'algorithme d'homologie locale de Neddleman et Wunsch (1970), au moyen de la méthode de recherche de similarité de Pearson et Lipman (1988), au moyen de logiciels informatiques utilisant ces algorithmes (GAP, BESTFIT, BLAST P, BLAST N, FASTA et TFASTA dans le Wisconsin Genetics Software Package, Genetics Computer Group, 575 Science Dr., Madison, WI). Afin d'obtenir l'alignement optimal, on utilise de préférence le programme BLAST, avec la matrice BLOSUM 62. On peut également utiliser les matrices PAM ou PAM250.

Le pourcentage d'identité entre deux séquences d'acides nucléiques ou d'acides aminés est déterminé en comparant ces deux séquences alignées de manière optimale, la séquence d'acides nucléiques ou d'acides aminés à comparer pouvant comprendre des additions ou des délétions par rapport à la séquence de référence pour un alignement optimal entre ces deux séquences. Le pourcentage d'identité est calculé en déterminant le nombre de positions identiques pour lesquelles le nucléotide ou le résidu d'acide aminé est identique entre les deux séquences, en divisant ce nombre de positions identiques par le nombre total de positions comparées et en multipliant le résultat obtenu par 100 pour obtenir le pourcentage d'identité entre ces deux séquences.

Par séquences nucléiques présentant un pourcentage d'identité d'au moins 80 %, de préférence 90 %, de façon plus préférée 98 %, après alignement optimal avec une séquence de référence, on entend désigner les séquences nucléiques présentant, par rapport à la séquence nucléique de référence, certaines modifications comme en particulier une délétion, une troncation, un allongement, une fusion chimérique, et/ou une substitution, notamment ponctuelle, et dont la séquence nucléique présente au moins 80 %, de préférence 90 %, de façon plus préférée 98 %, d'identité après alignement optimal avec la séquence nucléique de référence. Il s'agit de préférence de séquences dont les séquences complémentaires sont susceptibles de s'hybrider spécifiquement avec la séquence SEQ ID N° 1 de l'invention. De préférence, les conditions d'hybridation spécifiques ou de forte stringence seront telles qu'elles assurent au moins 80 %, de préférence 90 %, de façon plus préférée 98 % d'identité après alignement optimal entre l'une des deux séquences et la séquence complémentaire de l'autre.

Une hybridation dans des conditions de forte stringence signifie que les conditions de température et de force ionique sont choisies de telle manière qu'elles permettent le maintien de l'hybridation entre deux fragments d'ADN complémentaires. A titre illustratif, des conditions de forte stringence de l'étape d'hybridation aux fins de définir les fragments polynucléotidiques décrits ci-dessus, sont avantageusement les suivantes.

L'hybridation ADN-ADN ou ADN-ARN est réalisée en deux étapes : (1) préhybridation à 42°C pendant 3 heures en tampon phosphate (20 mM, pH 7,5) contenant 5 x SSC (1 x SSC correspond à une solution 0,15 M NaCl + 0,015 M citrate de sodium), 50 % de formamide, 7 % de sodium dodécyl sulfate (SDS), 10 x Denhardt's, 5 % de dextran sulfate et 1 % d'ADN de sperme de saumon ; (2) hybridation proprement dite pendant 20 heures à une température dépendant de la taille de la sonde (i.e. : 42°C, pour une sonde de taille > 100 nucléotides) suivie de 2 lavages de 20 minutes à 20°C en 2 x SSC + 2 % SDS, 1 lavage de 20 minutes à 20°C en 0,1 x SSC + 0,1 % SDS. Le dernier lavage est pratiqué en 0,1 x SSC + 0,1 % SDS pendant 30 minutes à 60°C pour une sonde de taille > 100 nucléotides. Les conditions d'hybridation de forte stringence décrites ci-dessus pour un polynucléotide de taille définie, peuvent être adaptées par l'homme du métier pour des oligonucléotides de taille plus grande ou plus petite, selon l'enseignement de Sambrook et al., 1989.

Parmi les séquences nucléiques présentant un pourcentage d'identité d'au moins 80 %, de préférence 90 %, de façon plus préférée 98 %, après alignement optimal avec la séquence selon l'invention, on préfère également les séquences nucléiques variantes de SEQ ID N° 1, ou de ses fragments, c'est-à-dire l'ensemble des séquences nucléiques correspondant à des variants alléliques, c'est-à-dire des variations individuelles de la séquence SEQ ID N° 1. Ces séquences mutées naturelles correspondent à des polymorphismes présents chez les oiseaux, en particulier chez les galliformes. De préférence, la présente invention concerne les séquences nucléiques variantes dans lesquelles les mutations conduisent à une modification de la séquence d'acides aminés du polypeptide, ou de ses fragments, codés par la séquence normale de SEQ ID N° 1.

On entend également désigner par séquence nucléique variante tout ARN ou ADNc résultant d'une mutation et/ou variation d'un site d'épissage de la séquence nucléique génomique dont l'ADNc a pour séquence SEQ ID N° 1.

L'invention concerne de préférence un acide nucléique purifié ou isolé selon la présente invention, caractérisé en ce qu'il comprend ou est constitué de la séquence SEQ ID N° 1, de sa séquence complémentaire ou de la séquence de l'ARN correspondant à SEQ ID N° 1.

Les amorces ou sondes, caractérisées en ce qu'elles comprennent une séquence d'un acide nucléique selon l'invention, font également partie de l'invention.

Ainsi, la présente invention concerne également les amorces ou les sondes selon l'invention qui peuvent permettre en particulier de mettre en évidence ou de discriminer les séquences nucléiques variantes, ou d'identifier la séquence gnomique du gène dont l'ADNc est représenté par SEQ ID N° 1, en utilisant notamment une méthode d'amplification telle que la méthode PCR, ou une méthode apparentée.

L'invention concerne également l'utilisation d'une séquence d'acide nucléique choisie parmi une séquence d'acide nucléique selon l'invention, un fragment d'au moins 15 nucléotides consécutifs de la séquence SEQ ID N°1 et une séquence nucléique s'hybridant dans des conditions de forte stringence avec l'une de ces séquences, comme sonde ou amorce, pour la détection, l'identification, le dosage et/ou l'amplification de séquences d'acide nucléique selon l'invention.

L'invention concerne également l'utilisation d'une séquence d'acide nucléique choisie parmi une séquence d'acide nucléique selon l'invention, un fragment d'au moins 15 nucléotides consécutifs de la séquence SEQ ID N°1 et une séquence nucléique s'hybridant dans des conditions de forte stringence avec l'une de ces séquences, comme oligonucléotide sens ou antisens spécifique d'une séquence d'acide nucléique selon l'invention.

Selon l'invention, les polynucléotides pouvant être utilisés comme sonde ou comme amorce dans des procédés de détection, d'identification, de dosage ou d'amplification de séquence nucléique, présentent une taille minimale de 15 bases, de préférence de 20 bases, ou mieux de 25 à 30 bases.

Les sondes et amorces selon l'invention peuvent être marquées directement ou indirectement par un composé radioactif ou non radioactif par des méthodes bien connues de l'homme du métier, afin d'obtenir un signal détectable et/ou quantifiable.

Les séquences de polynucléotides selon l'invention non marquées peuvent être utilisées directement comme sonde ou amorce.

Les séquences sont généralement marquées pour obtenir des séquences utilisables pour de nombreuses applications. Le marquage des amorces ou des sondes selon l'invention est réalisé par des éléments radioactifs ou par des molécules non radioactives.

Parmi les isotopes radioactifs utilisés, on peut citer le ³²P, le ³³P, le ³⁵5, le ³H ou le ¹²⁵I. Les entités non radioactives sont sélectionnées parmi les ligands tels la biotine, l'avidine, la streptavidine, la dioxygénine, les haptènes, les colorants, les agents luminescents tels que les agents radioluminescents, chémoluminescents, bioluminescents, fluorescents, phosphorescents.

Les polynucléotides selon l'invention peuvent ainsi être utilisés comme amorce et/ou sonde dans des procédés mettant en oeuvre notamment la technique de PCR (amplification en chaîne par polymérase) (Rolfs et al., 1991). Cette technique nécessite le choix de paires d'amorces oligonucléotidiques encadrant le fragment qui doit être amplifié. On peut, par exemple, se référer à la technique décrite dans le brevet américain U.S. N° 4,683,202. Les fragments amplifiés peuvent être identifiés, par exemple après une électrophorèse en gel d'agarose ou de polyacrylamide, ou après une technique chromatographique comme la filtration sur gel ou la chromatographie échangeuse d'ions, puis séquencés. La spécificité de l'amplification peut être contrôlée en utilisant comme amorces les séquences nucléotidiques de polynucléotides de l'invention et comme matrices, des plasmides contenant ces séquences ou encore les produits d'amplification dérivés. Les fragments nucléotidiques amplifiés peuvent être utilisés comme réactifs dans des réactions d'hybridation afin de mettre en évidence la présence, dans un échantillon biologique, d'un acide nucléique cible de séquence complémentaire à celle desdits fragments nucléotidiques amplifiés.

L'invention vise également les acides nucléiques susceptibles d'être obtenus par amplification à l'aide d'amorces selon l'invention.

D'autres techniques d'amplification de l'acide nucléique cible peuvent être avantageusement employées comme alternative à la PCR (PCR-like) à l'aide de couple d'amorces de séquences nucléotidiques selon l'invention. Par PCR-like on entend désigner toutes les méthodes mettant en oeuvre des reproductions directes ou indirectes des séquences d'acides nucléiques, ou bien dans lesquelles les systèmes de marquage ont été amplifiés, ces techniques sont bien entendu connues. En général il s'agit de l'amplification de l'ADN par une polymérase ; lorsque l'échantillon d'origine est un ARN il convient préalablement d'effectuer une transcription reverse. Il existe actuellement de très nombreux procédés permettant cette amplification, comme par exemple la technique SDA (Strand Displacement Amplification) ou technique d'amplification à déplacement de brin (Walker et al., 1992), la technique TAS (Transcription-based Amplification System) décrite par Kwoh et al. (1989), la technique 3SR (Self-Sustained Sequence Replication) décrite par Guatelli et al. (1990), la technique NASBA (Nucleic Acid Sequence Based Amplification) décrite par Kievitis et al. (1991), la technique TMA (Transcription Mediated Amplification), la technique LCR (Ligase Chain Reaction) décrite par Landegren et al. (1988), la technique de RCR (Repair Chain Reaction) décrite par Segev (1992), la technique CPR (Cycling Probe Reaction) décrite par Duck et al. (1990), la technique d'amplification à la Q-béta-réplicase décrite par Miele et al. (1983). Certaines de ces techniques ont depuis été perfectionnées.

Dans le cas où le polynucléotide cible à détecter est un ARNm, on utilise avantageusement, préalablement à la mise en oeuvre d'une réaction d'amplification à l'aide des amorces selon l'invention ou à la mise en oeuvre d'un procédé de détection à l'aide des sondes de l'invention, une enzyme de type transcriptase inverse afin d'obtenir un ADNc à partir de l'ARNm contenu dans l'échantillon biologique. L'ADNc obtenu servira alors de cible pour les amorces ou les sondes mises en oeuvre dans le procédé d'amplification ou de détection selon l'invention.

La technique d'hybridation de sondes peut être réalisée de manières diverses (Matthews et al., 1988). La méthode la plus générale consiste à immobiliser l'acide nucléique extrait des cellules de différents tissus ou de cellules en culture sur un support (tels que la nitrocellulose, le nylon, le polystyrène) et à incuber, dans des conditions bien définies, l'acide nucléique cible immobilisé avec la sonde. Après l'hybridation, l'excès de sonde est éliminé et les molécules hybrides formées sont détectées par la méthode appropriée (mesure de la radioactivité, de la fluorescence ou de l'activité enzymatique liée à la sonde).

Selon un autre mode de mise en oeuvre des sondes nucléiques selon l'invention, ces dernières peuvent être utilisées comme sondes de capture. Dans ce cas, une sonde, dite « sonde de capture », est immobilisée sur un support et sert à capturer par hybridation spécifique l'acide nucléique cible obtenu à partir de l'échantillon biologique à tester et l'acide nucléique cible est ensuite détecte grâce à une seconde sonde, dite « sonde de détection », marquée par un élément facilement détectable.

Parmi les fragments d'acides nucléiques intéressants, il faut ainsi citer en particulier les oligonucléotides anti-sens, c'est-à-dire dont la structure assure, par hybridation avec la séquence cible, une inhibition de l'expression du produit correspondant. Il faut également citer les oligonucléotides sens qui, par interaction avec des protéines impliquées dans la régulation de l'expression du produit correspondant, induiront soit une inhibition, soit une activation de cette expression.

Dans un mode de réalisation particulier de l'invention, l'acide nucléique selon l'invention code pour un polypeptide possédant un fragment continu d'au moins 200 acides aminés de la protéine SEQ ID N° 2, de préférence 300 acides aminés, de la façon la plus préférée pour la protéine SEQ ID N° 2. Ce polypeptide est également un objet de l'invention.

En effet, la présente invention concerne également un polypeptide isolé caractérisé en ce qu'il comprend un polypeptide choisi parmi:
a) un polypeptide de séquence SEQ ID N° 2 ;
b) un polypeptide comportant au moins 80 % d'identité avec ledit polypeptide de a), possédant un rôle dans le caractère de pluripotence des cellules ES et utilisable comme marqueur du caractère de pluripotence des cellules ES.

De préférence, l'acide aminé en position 455 est une thréonine.

Par « polypeptide », on entend, au sens de la présente invention, désigner des protéines ou des peptides.

De préférence un polypeptide selon l'invention est un polypeptide constitué de la séquence SEQ ID N° 2 (correspondant à la protéine codée par le gène *ens-1)* ou d'une séquence possédant au moins 80 % d'identité avec SEQ ID N° 2 après alignement optimal.

La séquence du polypeptide présente un pourcentage d'identité d'au moins 80 % après alignement optimal avec la séquence SEQ ID N° 2, de préférence 90 %, de façon plus préférée 98 %.

Par polypeptide dont la séquence d'acides aminés présentant un pourcentage d'identité d'au moins 80 %, de préférence 90 %, de façon plus préférée 98 %, après alignement optimal avec une séquence de référence, on entend désigner les polypeptides présentant certaines modifications par rapport au polypeptide de référence, comme en particulier une ou plusieurs délétions, troncations, un allongement, une fusion chimérique, et/ou une ou plusieurs substitutions.

Parmi les polypeptides dont la séquence d'acides aminés présentant un pourcentage d'identité d'au moins 80 %, de préférence 90 %, de façon plus préférée 98 %, après alignement optimal avec la séquence SEQ ID N° 2 ou avec l'un de ses fragments selon l'invention, on préfère les polypeptides variants codés par les séquences nucléiques variantes telles que précédemment définies, en particulier les polypeptides dont la séquence d'acides aminés présente au moins une mutation correspondant notamment à une troncation, délétion, substitution et/ou addition d'au moins un résidu d'acide aminé par rapport à la séquence SEQ ID N° 2 ou avec l'un de ses fragments, de manière plus préférée les polypeptides variants présentant une mutation liée à une perte de caractère pluripotent des cellules les contenant.

La présente invention concerne également les vecteurs de clonage et/ou d'expression comprenant un acide nucléique ou codant pour un polypeptide selon l'invention. Un tel vecteur peut également contenir les éléments nécessaires à l'expression et éventuellement à la sécretion du polypeptide dans une cellule hôte. Une telle cellule hôte est également un objet de l'invention.

Les vecteurs caractérisés en ce qu'ils comportent une séquence de promoteur et/ou de régulateur selon l'invention, font également partie de l'invention.

Lesdits vecteurs comportent de préférence un promoteur, des signaux d'initiation et de terminaison de la traduction, ainsi que des régions appropriées de régulation de la transcription. Ils doivent pouvoir être maintenus de façon stable dans la cellule et peuvent éventuellement posséder des signaux particuliers spécifiant la sécrétion de la protéine traduite.

Ces différents signaux de contrôle sont choisis en fonction de l'hôte cellulaire utilisé. A cet effet, les séquences d'acide nucléique selon l'invention peuvent être insérées dans des vecteurs à réplication autonome au sein de l'hôte choisi, ou des vecteurs intégratifs de l'hôte choisi.

Parmi les systèmes à réplication autonome, on utilise de préférence en fonction de la cellule hôte, des systèmes de type plasmidique ou viral, les vecteurs viraux pouvant notamment être des adénovirus (Perricaudet et al., 1992), des rétrovirus, des lentivirus, des poxvirus ou des virus herpétiques (Epstein et al., 1992). L'homme du métier connaît les technologies utilisables pour chacun de ces systèmes.

Lorsque l'on souhaite l'intégration de la séquence dans les chromosomes de la cellule hôte, on peut utiliser par exemple des systèmes de type plasmidique ou viral ; de tels virus sont, par exemple, les rétrovirus (Temin, 1986), ou les AAV (Carter, 1993).

Parmi les vecteurs non viraux, on préfère les polynucléotides nus tels que l'ADN nu ou l'ARN nu selon la technique développée par la société VICAL, les chromosomes artificiels de bactérie (BAC, bacterial artificial chromosome), les chromosomes artificiels de levure (YAC, yeast artificial chromosome) pour l'expression dans la levure, les chromosomes artificiels de souris (MAC, mouse artificial chromosome) pour l'expression dans les cellules murines et de manière préférée les chromosomes artificiels d'homme (HAC, human artificial chromosome) pour l'expression dans les cellules humaines.

Dans les cellules d'oiseaux, on pourra utiliser comme vecteur d'expression des retrovirus, des adénovirus aviaires, des poxvirus ou bien de l'ADN introduit par transfection ou électroporation.

De tels vecteurs sont préparées selon les méthodes couramment utilisées par l'homme du métier, et les clones en résultant peuvent être introduits dans un hôte approprié par des méthodes standard, telles que par exemple la lipofection, l'électroporation, le choc thermique, la transformation après perméabilisation chimique de la membrane, la fusion cellulaire.

L'invention comprend en outre les cellules hôtes, notamment les cellules eucaryotes et procaryotes, à l'exception des cellules ES humaines, transfomées par les vecteurs selon l'invention ainsi que les animaux transgéniques, de préférence les oiseaux ou mammifères, excepté l'Homme, comprenant une desdites cellules transformées selon l'invention. En particulier, l'invention comprend les animaux comprenant le gène *ens-1* présentant des marqueurs génétiques insérés dans celui-ci.

Parmi les cellules utilisables aux sens de la présente invention, on peut citer les cellules bactériennes (Olins et Lee, 1993), mais aussi les cellules de levure (Buckholz, 1993), de même que les cellules animales, en particulier les cultures de cellules de mammifères (Edwards et Aruffo, 1993), et notamment les cellules d'ovaire de hamster chinois (CHO). On peut citer également les cellules d'insectes dans lesquelles on peut utiliser des procédés mettant par exemple en oeuvre des baculovirus (Luckow, 1993). Un hôte cellulaire préféré pour l'expression des protéines de l'invention est constitué par les cellules COS.

Parmi les cellules d'oiseaux utilisables, on peut citer les cellules LMH d'hématome de poulet, les cellules immortalisées de caille QT6, les fibroblastes primaires ou immortalisés de poulet, de caille, de canard.

L'invention concerne également une cellule hôte contenant un acide nucléique selon l'invention, caractérisée en ce qu'il s'agit d'une cellule ES d'oiseau modifiée en outre par introduction d'un gène exogène, ledit gène exogène étant intégré dans ledit acide nucleique selon l'invention et étant uniquement et spécifiquement exprimé lorsque ladite cellule est maintenue à l'état pluripotent. De façon préférée, ledit gène exogène est un gène rapporteur, choisi parmi lacZ GFP, luciférase, ROSA-β-geo, un gène de résistance à un antibiotique en particulier les gens de résistance à la néomycine, l'hygromycine, la phléomycine, la puromycine).

Ces cellules selon l'invention sont très utiles pour le criblage de composés permettant d'induire la différentiation de cellules pluripotentes, ou de milieu pour la culture de cellules tout en maintenant leur caractère pluripotent.

Une autre cellule hôte d'intérêt selon l'invention consiste en une cellule d'oiseau contenant un acide nucléique selon l'invention, modifiée en outre par introduction d'un acide nucléique exogène, ledit acide nucléique exogène étant intégré dans ledit acide nucléique selon l'invention. Selon un mode de réalisation préférée de l'invention, ledit acide nucléique exogène est un gène d'intérêt thérapeutique, éventuellement précédé d'un promoteur spatio-temporel et/ou de séquences terminatrices. Dans un autre mode de réalisation, ledit acide nucléique exogène est un marqueur génétique, qui peut être choisi parmi lacZ, GFP, phosphatase alcaline, thymidine kinase, gènes de résistance aux antibiotiques (parmi lesquels néomycine, hygromycine, phléomycine, puromycine).

De façon préférée, les cellules d'oiseau hôtes précédemment décrites sont caractérisées en ce que ledit oiseau appartient à l'ordre des Galliformes et est en particulier un poulet ou une caille.

Dans ce cas, on intégre ledit gène rapporteur sous le contrôle du promoteur du gène *ens-1* de sequence nucléotidique 3111-3670 de SEQ IDN°1 et/ou on intègre ledit acide nucléique exogène (gène d'intérêt thérapeutique et/ou marqueur génétique) dans le gène *ens*-*1*.

On peut également modifier ce promoteur, en réduisant le nombre de nucléotides, ou en en introduisant de nouveaux, voire en effectuant des mutations sur certains nucléotides. L'homme du métier connaît les protocoles pour effectuer de telles modifications, ainsi que pour tester le promoteur ainsi obtenu pour l'expression dans les cellules souches pluripotentes. On a ainsi montré en particulier que l'on peut insérer une guanine en position 3654 de SEQ ID N° 1, sans perdre l'activité promotrice du fragment ainsi modifié.

Ainsi, l'invention concerne aussi l'utilisation d'un acide nucléique correspondant aux nucléotides 3111-3670 de SEQ ID N° 1 en tant que promoteur d'un gène d'intérêt pour une expression spécifique dudit gène d'intérêt dans des cellules pluripotentes aviaires. Un gène d'intérêt est soit un gène marqueur (luciférase, GFP, β-galactosidase...), soit peut être un gène codant pour une protéine telle un facteur de croissance, une cytokine, une protéine impliquée dans la reconnaissance immunitaire, une protéine à intérêt thérapeutique... Il est intéressant de noter que la « TATA box » a également été identifiée, qui est un objet de l'invention, aux nucléotides 3645-3651 de SEQ ID N° 1.

Une cellule préférée selon l'invention est une cellule 9N2.5, déposée à la Collection Nationale de Culture des Microorganismes le 11 Mai 2000 sous le numéro d'ordre 1-2477.

Les cellules selon l'invention sont de préférence des cellules ES pluripotentes, mais il doit être compris que l'invention concerne aussi les cellules d'oiseau différentiées, dérivant d'une cellule ES selon l'invention. on peut en particulier effectuer la différentiation de ces cellules en utilisant de l'acide rétinoique, selon les enseignements de la demande de brevet WO 96/12793.

L'invention concerne également les animaux transgéniques, excepté l'homme, qui contiennent une cellule selon l'invention. Parmi les animaux selon l'invention, on préfère les oiseaux, en particulier les membres de l'ordre des Galliformes. Ces oiseaux transgéniques seront particulièrement intéressants pour l'étude de modifications dans le gène *ens-1* ou dans son promoteur.

On peut également introduire un acide nucléique selon l'invention dans des oiseaux et d'autres animaux tels que les rongeurs, en particulier les souris, les rats ou les lapins, afin d'exprimer un polypeptide selon l'invention.

Ces animaux transgéniques sont obtenus par exemple par recombinaison homologue sur cellules souches embryonnaires, transfert de ces cellules souches à des embryons, sélection des chimères affectées au niveau des lignées reproductrices, et croissance desdites chimères. Ils peuvent aussi être obtenus par microinjection d'ADN nu dans l'ovocyte fécondé.

Les animaux transgéniques selon l'invention peuvent ainsi surexprimer le gène codant pour la protéine selon l'invention, ou leur gène homologue, ou exprimer ledit gène dans lequel est introduite une mutation ou bien exprimer un transgène comportant des portions du gène *ens-1* associées à des séquences codantes destinées à produire une protéine.

Alternativement, les oiseaux transgéniques selon l'invention peuvent être rendus déficients pour le gène codant pour le polypeptide de séquence SEQ ID N° 2, ou un gènes homologues, par inactivation à l'aide du système LOXP/CRE recombinase (Rohlmann et al., 1996) ou de tout autre système d'inactivation de l'expression de ce gène.

L'invention concerne aussi l'utilisation d'une séquence d'acide nucléique selon l'invention pour la synthèse de polypeptides recombinants selon l'invention.

La méthode de production d'un polypeptide de l'invention sous forme recombinante, elle-même comprise dans la présente invention, se caractérise en ce que l'on cultive les cellules transformées, notamment les cellules ou mammifères de la présente invention, dans des conditions permettant l'expression d'un polypeptide recombinant codé par une séquence d'acide nucléique selon l'invention, et que l'on récupère ledit polypeptide recombinant.

Les polypeptides recombinants, caractérisés en ce qu'ils sont susceptibles d'être obtenus par ladite méthode de production, font également partie de l'invention.

Les polypeptides recombinants obtenus comme indiqué ci-dessus, peuvent aussi bien se présenter sous forme glycosylée que non glycosylée et peuvent présenter ou non la structure tertiaire naturelle.

Les séquences des polypeptides recombinants peuvent être également modifiées afin d'améliorer leur solubilité, en particulier dans les solvants aqueux.

De telles modifications sont connues de l'homme du métier comme par exemple la délétion de domaines hydrophobes ou la substitution d'acides aminés hydrophobes par des acides aminés hydrophiles.

Ces polypeptides peuvent être produits à partir des séquences d'acide nucléique définies ci-dessus, selon les techniques de production de polypeptides recombinants connues de l'homme du métier. Dans ce cas, la séquence d'acide nucléique utilisée est placée sous le contrôle de signaux permettant son expression dans un hôte cellulaire.

Un système efficace de production d'un polypeptide recombinant nécessite de disposer d'un vecteur et d'une cellule hôte selon l'invention.

Ces cellules peuvent être obtenues par l'introduction dans des cellules hôtes d'une séquence nucléotidique insérée dans un vecteur tel que défini ci-dessus, puis la mise en culture desdites cellules dans des conditions permettant la réplication et/ou l'expression de la séquence nucléotidique transfectée.

Les procédés utilisés pour la purification d'un polypeptide recombinant sont connus de l'homme du métier. Le polypeptide recombinant peut être purifié à partir de lysats et extraits cellulaires, du surnageant du milieu de culture, par des méthodes utilisées individuellement ou en combinaison, telles que le fractionnement, les méthodes de chromatographie, les techniques d'immunoaffinité à l'aide d'anticorps monoclonaux ou polyclonaux spécifiques, etc...

Les polypeptides selon la présente invention peuvent aussi être obtenus par synthèse chimique en utilisant l'une des nombreuses synthèses peptidiques connues, par exemple les techniques mettant en oeuvre des phases solides (voir notamment Stewart et al., 1984) ou des techniques utilisant des phases solides partielles, par condensation de fragments ou par une synthèse en solution classique.

Les polypeptides obtenus par synthèse chimique et pouvant comporter des acides aminés non naturels correspondants sont également compris dans l'invention.

Les anticorps mono- ou polyclonaux ou leurs fragments, anticorps chimériques ou immunoconjugués, caractérisés en ce qu'ils sont capables de reconnaître spécifiquement un polypeptide selon l'invention, font partie de l'invention.

Des anticorps polyclonaux spécifiques peuvent être obtenus à partir d'un sérum d'un animal immunisé contre les polypeptides selon l'invention, notamment produit par recombinaison génétique ou par synthèse peptidique, selon les modes opératoires usuels.

On note notamment l'intérêt d'anticorps reconnaissant de façon spécifique certains polypeptides, variants, ou leurs fragments immunogènes, selon l'invention.

Les anticorps mono- ou polyclonaux ou leurs fragments, anticorps chimériques ou immunoconjugués, caractérisés en ce qu'ils sont capables de reconnaître spécifiquement le polypeptide de séquence SEQ ID N° 2 sont particulièrement préférés.

Les anticorps monoclonaux spécifiques peuvent être obtenus selon la méthode classique de culture d'hybridomes décrite par Köhler et Milstein (1975).

Les anticorps selon l'invention sont, par exemple, des anticorps chimériques, des anticorps humanisés, des fragments Fab ou F(ab')₂. Ils peuvent également se présenter sous forme d'immunoconjugués ou d'anticorps marqués afin d'obtenir un signal détectable et/ou quantifiable.

L'invention concerne également des méthodes pour la détection et/ou la purification d'un polypeptide selon l'invention, caractérisées en ce qu'elles mettent en oeuvre un anticorps selon l'invention.

L'invention comprend en outre des polypeptides purifiés, caractérisés en ce qu'ils sont obtenus par une méthode selon l'invention.

Par ailleurs, outre leur utilisation pour la purification des polypeptides, les anticorps de l'invention, en particulier les anticorps monoclonaux, peuvent également être utilisés pour la détection de ces polypeptides dans un échantillon biologique.

Ils constituent ainsi un moyen d'analyse immunocytochimique ou immunohistochimique de l'expression des polypeptides selon l'invention, notamment le polypeptide de séquence SEQ ID N° 2 ou l'un de ses variants, sur des coupes de tissus spécifiques, par exemple par immunofluorescence, marquage à l'or, immunoconjugués enzymatiques.

Ils peuvent permettre notamment de mettre en évidence l'expression de ces polypeptides dans les tissus ou prélèvements biologiques.

Plus généralement, les anticorps de l'invention peuvent être avantageusement mis en oeuvre dans toute situation où l'expression d'un polypeptide selon l'invention, normal ou muté, doit être observée.

Ainsi, un procédé de détection d'un polypeptide selon l'invention dans un échantillon biologique, comprenant les étapes de mise en contact de l'échantillon biologique avec un anticorps selon l'invention et de mise en évidence du complexe antigène-anticorps formé est également un objet de l'invention, ainsi qu'une trousse permettant de mettre en oeuvre un tel procédé. Une telle trousse contient en particulier :
a) un anticorps monoclonal ou polyclonal selon l'invention ;
b) éventuellement des réactifs pour la constitution d'un milieu propice à la réaction immunologique ;
c) les réactifs permettant la détection du complexe antigène-anticorps produit lors de la réaction immunologique.

Ces anticorps peuvent être obtenus directement à partir de sérum humain, ou à partir d'animaux immunisés avec des polypeptides selon l'invention, puis « humanisés ».

Les anticorps selon l'invention sont très utiles pour déterminer la présence du polypeptide SEQ ID N° 2, et permettent ainsi de déterminer le caractère pluripotent d'une cellule ES d'oiseau.

Un procédé de détermination du caractère pluripotent d'une cellule ES d'oiseau, caractérisé en ce qu'on détermine la présence d'un produit d'expression du gène correspondant à SEQ ID N° 1 ou de l'ARNm de SEQ ID N° 1 est également un objet l'invention.

En effet, l'invention divulgue la séquence du gène *ens-1,* qui est spécifiquement exprimé dans les cellules ES d'oiseau, en particulier des Galliformes, lorsque celles-ci sont pluripotentes. Les méthodes de détection de l'expression d'un gène appliquées à ce gène permettent donc de connaître rapidement la nature des cellules étudiées.

En particulier, comme écrit ci-dessus, on peut détecter le produit d'expression du gène, en utilisant par exemple des anticorps selon l'invention, par Western Blot ou d'autres méthodes décrites précédemment.

On peut aussi effectuer la détection de l'ARNm de SEQ ID N° 1 par Northern Blot ou par RT-PCR en utilisant une sonde ou des amorces selon l'invention.

La détection de l'expression de ce gène peut également être effectuée en utilisant une puce à ADN ou une puce à protéine, qui contiennent respectivement un acide nucléique ou un polypeptide selon l'invention. De telles puces sont également objets de l'invention.

Une puce à protéines selon l'invention permet aussi l'étude des interactions entre les polypeptides selon l'invention et d'autres protéines ou des composés chimiques, et peut ainsi être utile pour le criblage de composés interagissant avec les polypeptides selon l'invention.

Les Demanderesses ont montré que le gène *ens-1* est trouvé uniquement dans les oiseaux de la famille des Galliformes. Ainsi, l'invention concerne également un procédé de classification de l'appartenance d'un oiseau à l'ordre des Galliformes, caractérisé en ce que l'on détecte, dans le génome dudit oiseau, la présence d'un acide nucléique choisi parmi un acide nucléique selon l'invention, en particulier SEQ ID N° 1, un fragment d'au moins 15 nucléotides consécutifs de la séquence SEQ ID N° 1 et une séquence nucléique s'hybridant dans des conditions de forte stringence avec l'une de ces séquences.

Cette propriété que le gène *ens-1* soit trouvé uniquement chez les Galliformes permet de définir un procédé de détermination de la présence d'un échantillon provenant d'un oiseau de l'ordre des Galliformes dans un échantillon alimentaire, caractérisé en ce que l'on détecte, dans ledit échantillon, la présence d'un acide nucléique choisi parmi un acide nucléique selon l'invention, en particulier SEQ ID N° 1, un fragment d'au moins 15 nucléotides consécutifs de la séquence SEQ ID N° 1 et une séquence nucléique s'hybridant dans des conditions de forte stringence avec l'une de ces séquences.

On peut détecter la présence d'un acide nucléique selon l'invention dans un échantillon biologique ou alimentaire, ou dans le génome d'un oiseau, par différentes manières. En particulier, on peut définir un procédé de détection et/ou de dosage d'un acide nucléique selon l'invention dans un échantillon biologique, alimentaire caractérisé en ce qu'il comprend les étapes suivantes :
a) mise en contact dudit échantillon avec un polynucléotide marqué choisi parmi un polynucléotide selon l'invention, un fragment d'au moins 15 nucléotides consécutifs de la séquence SEQ ID N°1 et une séquence nucléique s'hybridant dans des conditions de forte stringence avec l'une de ces séquences ;
b) détection et/ou dosage de l'hybride formé entre ledit polynucléotide et l'acide nucléique dudit échantillon.

On peut également procéder à la détection et/ou au dosage d'un acide nucléique selon l'invention dans un échantillon biologique ou alimentaire, en effectuant une étape d'amplification des acides nucléiques dudit échantillon à l'aide d'amorces choisies parmi les acides nucléiques selon l'invention, un fragment d'au moins 15 nucléotides consécutifs de la séquence SEQ ID N° 1 et une séquence nucléique s'hybridant dans des conditions de forte stringence avec l'une de ces séquences.

Ainsi qu'il est démontré dans les exemples, l'acide nucléique selon l'invention n'est exprimé dans les cellules ES d'oiseau que lorsque celles-ci possèdent un caractère pluripotent. Par ailleurs, les cellules ES modifiées selon l'invention, avec un gène rapporteur exprimé spécifiquement lorsqu'elles sont pluripotentes, et en particulier les cellules 9N2.5, peuvent être utilisées pour cribler des composés d'intérêt.

En particulier, elles peuvent être utilisées dans un procédé de criblage d'une substance ou d'un milieu capables d'induire une différentiation de cellules pluripotentes, caractérisé en ce que qu'il comprend les étapes suivantes :
a) maintien de cellules ES, à l'exception des cellules ES humaines, selon l'invention dans un milieu de culture permettant le maintien du phénotype pluripotent ;
b) ajout de ladite substance dans ledit milieu de culture ou remplacement dudit milieu de culture par le milieu à tester ;
c) détermination de l'induction de la différentiation par l'absence d'expression de la protéine SEQ ID N° 2 ou du gène exogène.

Ce procédé est de préférence mis en oeuvre avec des cellules ES modifiées par insertion d'un gène rapporteur sous le contrôle du promoteur du gène *ens-1,* et l'on détecte l'absence d'expression dudit gène rapporteur. On utilise de préférence les cellules 9N2.5, et l'on détecte l'absence d'expression de la β-galactosidase.

On peut également utiliser les cellules selon l'invention pour cribler des substances capables de restaurer le caractère pluripotent de cellules différentiées dérivant de cellules ES d'oiseau selon l'invention, modifiées en outre par introduction d'un gène exogène, ledit gène exogène étant intégré dans l'acide nucléique selon l'invention et étant uniquement et spécifiquement exprimé lorsque ladite cellule est maintenue à l'état pluripotent, par un procédé comprenant les étapes suivantes:
a) maintien desdites cellules différentiées dans un milieu de culture adéquat ;
b) remplacement dudit milieu de culture par un milieu permettant de maintenir un phénotype pluripotent, et contenant ladite substance à tester ;
c) détermination de la restauration du caractère pluripotent desdites cellules par l'expression de la protéine SEQ ID N° 2 ou du gène exogène, dans lesdites cellules.

Ce procédé est de nouveau avantageusement mise en oeuvre avec des cellules différentiées selon l'invention, modifiées par insertion d'un gène rapporteur dans le gène *ens-l* ou sous contrôle de son promoteur. On utilise avantageusement des cellules 9N2.5 différentiées, qui permettent la détection de l'expression de la β-galactosidase.

Les procédés décrits ci-dessus sont également objets de l'invention, ainsi que les milieux ou substances obtenus par lesdits procédés.

Une telle substance selon l'invention peut être un composé ayant une structure chimique (du type petite molécule organique), un lipide, un sucre, une protéine, un peptide, un composé hybride protéine-lipide, protéine-sucre, peptide-lipide, ou peptide-sucre, une protéine ou un peptide sur lequel on a ajouté des ramifications chimiques.

Parmi les composés chimiques envisagés, ils peuvent contenir un ou plusieurs cycles, aromatique(s) ou non, ainsi que plusieurs résidus de toute sorte (notamment alkyle inférieur, c'est-à-dire présentant entre 1 à 6 atomes de carbones).

Il est extrêmement important de déterminer les gènes impliqués dans le caractère de pluripotence des cellules ES, ou de bénéficier d'un marqueur dudit caractère. En effet, du fait de la capacité pour ces cellules de participer à la morphogenèse de tous les tissus, une modification génétique de celles-ci permet d'assurer que le caractère recherché se retrouvera dans tous les tissus de l'animal formé. Par ailleurs, l'introduction de gènes exogènes au locus du gène *ens-1*, sous le contrôle de promoteurs à spécificité spatio-temporelle variables peut permettre l'obtention d'animaux transgéniques exprimant lesdits gènes dans des tissus ou à des stades de développement donnés. En effet, la spécificité du gène *ens-1* étant qu'il ne s'exprime que si la cellule hôte possède le caractère pluripotent, l'introduction d'un acide nucléique exogène dans ce locus ne devrait pas gêner le développement de l'embryon.

On peut donc introduire des gènes d'intérêt thérapeutique, par exemple codant pour des protéines thérapeutiques (hormones, facteurs de croissance, lymphokines), afin de pouvoir produire ces protéines lors du développement de l'embryon. Il peut en effet être très intéressant de produire des protéines thérapeutiques dans les oeufs, dont la coquille assure un environnement stérile.

On peut également utiliser des cellules pluripotentes selon l'invention afin de les faire coloniser le tissu germinal d'animaux, en particulier d'oiseaux, de façon plus préférée de l'ordre des Galliformes, afin que des caractères génétiques particuliers puissent être transmis à leur descendance. Ceci permet l'amélioration de races industrielles de poulets, dindes, cailles ou autres d'une façon particulièrement intéressante économiquement.
On peut aussi utiliser les composés choisis parmi
a) un acide nucléique selon l'invention ;
b) un polypeptide selon l'invention ;
c) un vecteur selon l'invention ;
d) une cellule selon l'invention ;
e) un anticorps selon l'invention ;
   à titre de médicament, afin de permettre, selon le cas, la restauration du caractère pluripotent de cellules d'oiseaux, ou au contraire d'induire la différentiation de cellules ES.

La présente invention ouvre donc la voie à une meilleure caractérisation du caractère pluripotent des cellules ES, en fournissant la séquence d'un marqueur de ces cellules. Il reste toutefois à déterminer si ce gène est un facteur essentiel de ce caractère. Ainsi, l'introduction du gène *ens-1* dans des cellules différentiées, par exemple sur un plasmide sous le contrôle d'un promoteur adapté, et l'étude de la restauration éventuelle du caractère pluripotent de ces cellules permettra de répondre à cette question. Parmi les promoteurs adaptés, on choisira un promoteur inductible, par exemple à un sucre, et l'on déterminera le caractère pluripotent des cellules lorsque l'on cesse d'induire l'expression du gène sur le plasmide. On peut également construire un plasmide qui mène à une excision du gène *ens-1* après un certain temps (par exemple en le plaçant entre deux séquences loxP, et en introduisant un second plasmide codant pour la recombinase Cre). Pour déterminer le caractère pluripotent des cellules, il peut être avantageux d'utiliser les cellules 9N2.5 selon l'invention, et de rechercher l'expression de la β-galactosidase après introduction du plasmide codant pour *ens-1.*

S'il est possible de déterminer que le gène *ens-1* est inducteur du caractère pluripotent des cellules, on peut mettre en oeuvre un procédé de restauration dudit caractère (également objet de l'invention), caractérisé en ce que l'on exprime le gène *ens-1* dans des cellules différentiées. On peut utiliser les méthodes décrites ci-dessus, en y apportant certaines améliorations connues de l'homme du métier.

Les exemples ci-dessous permettent d'illustrer l'invention, et ne doivent pas être considérés comme limitant l'invention.

### DESCRIPTION DES FIGURES

Figure 1 : structure du vecteur ROSA-β-geo utilisé pour transformer les cellules ES.
Figure 2 : analyse de l'expression du transcrit ROSA-β-geo par RT-PCR dans les cellules 9N2.5 lors de l'induction de la différentiation par l'acide rétinoïque (+RA), le DMSO (+DMSO) ou les deux simultanément (+RA+DMSO). Le milieu contrôle ne contient aucun facteur inducteur.
Figure 3 : analyse de l'expression du transcrit ROSA-β-geo par Northern Blot lors de l'induction de la différentiation par l'acide rétinoïque. Le Blot est hybridé avec une sonde LacZ.
Figure 4 : analyse de l'expression du transgène ROSA-β-geo par révélation de l'activité β-galactosidase dans des embryons chimères pour les cellules 9N2.5.
Figure 5 : analyse par PCR de la présence du transgène ROSA-β-geo dans les embryons chimères. De l'ADN a été extrait, soit de cellules 9N2.5, soit d'embryon chimère âgé de 48 heures ou de 4 jours, résultant de la transplantation de cellules 9N2.5, soit d'embryon contrôle de 48 heures ou 4 jours.
Figure 6 : détection par Southem Blot de la présence du transgène ROSA-β-geo dans l'ADN génomique des cellules 9N2.5 après digestion par *Eco*RI (E) ou *Dra*I (D).
Figure 7 : détection par Northern Blot de la présence d'un transcrit comportant le transgène ROSA-β-geo, par hybridation avec une sonde Lac Z.
Figure 8 : A. analyse par Northern Blot de l'expression du gène *ens-1* dans des cellules ES normales de poulet et dans les cellules 9N2.5, après hybridation par les sondes C1, S1 et S2. B. structure de l'ADN complémentaire du gène *ens-1.* (RS = séquences répétées, ORF = cadre de lecture). Les flèches représentent les sondes C1, S1 et S2 utilisées pour l'hybridation.
Figure 9 : analyse par Northern Blot de l'expression des transcrits *ens-1* dans les cellules souches embryonnaires normales de poulet, dans les cellules 9N2.5, dans l'embryon de poulet à différents stades de développement et dans différents organes de poussin. Les ARN polyA+ isolés à partir des ARN totaux ont été hybridés sur les blots avec les sondes C1 ou S1, ou avec une sonde contrôle GAPDH.
Figure 10 : analyse de l'expression du transcrit *ens-1* dans l'embryon de poulet par hybridation *in situ.*
Figure 11 : Amplification par PCR réalisé sur l'ADN génomique de différentes espèces aviaires avec les amorces ens1 S1 (SEQ ID N° 14) et ens1 AS1 (SEQ ID
N° 15).
Figure 12 : schéma de l'organisation des LTR de rétrovirus, de l'oragnisation attendue pour le gène ens-1, ainsi que des deux constructions utilisées pour l'identification du promoteur.
Figure 13 : Activité des promoteurs dans différentes lignées cellulaires (S : promoteur sens, AS : promoteur antisens)
Figure 14 : activité du promoteur 2 (figure 12) au cours de la différentiation des cellules ES.

### EXEMPLES

### Exemple 1 : Construction d'une cellule ES de poulet renfermant un marqueur génétique de la pluripotence.

Afin d'identifier un gène spécifiquement exprimé dans les cellules ES pluripotentes la stratégie dite de « piégeage de gène » (*gene trap*) a été suivie. Cette stratégie consiste à introduire, dans le génome des cellules ES, un gène marqueur qui comporte une séquence codante exogène mais qui est dépourvu de promoteur propre. L'insertion aléatoire de ce marqueur dans le génome de la cellule conduira dans certains cas à placer ce gène exogène en aval d'un promoteur propre du génome cellulaire. Le gène exogène adopte, dans cette configuration, une régulation d'expression très similaire sinon identique à celle du gène dans lequel il est inséré. Le suivi de l'expression du gène marqueur dans les cellules ainsi modifiées renseigne alors sur le patron d'expression du gène cellulaire ainsi « marqué ».

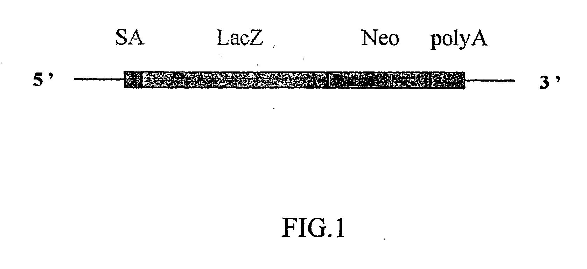

Comme système de piégeage de gène les inventeurs ont utilisé celui qui exploite les propriétés du vecteur ROSA-β-geo décrit par Friedrich et Soriano (1991). Ce système est constitué d'un plasmide qui porte les deux gènes respectivement LacZ et Neo^{R} fusionnés l'un à l'autre dans l'ordre 5'-3'. Ce gène fusion code pour une protéine unique LacZ-Neo qui confère aux cellules qui la produisent à la fois la résistance au G418 et l'activité β-galactosidase. La structure du plasmide est présentée sur la figure 1. Ce plasmide a été coupé par l'enzyme *Dra*I qui induit sa linéarisation. Le plasmide linéarisé a été introduit dans des cellules ES de poulet par la technique d'électroporation. Pour cela une culture de cellules ES de poulet entretenue dans les conditions décrites dans Pain et col. (1996) a été utilisée. Les cellules ES furent recueillies à partir de boîtes de culture par traitement ménagé à la pronase. Les cellules en suspension ont été lavées et mises en suspension dans du milieu de Glasgow à la concentration de 5 x 10⁶ dans 0,8 ml. Dix microgrammes de plasmide linéarisé ont été ajoutés à la suspension cellulaire qui a été maintenue pendant 10 minutes à 4°C. Ensuite la suspension a été soumise à un traitement d'électroporation consistant en 2 stimulations électriques dans les conditions suivantes: 280 V, 500 mF dans une cuve de 1 mm d'épaisseur dans un appareil *BioRad electroporator.* Les cellules ont ensuite été maintenues pendant 10 minutes à 4°C avant d'être ensemencées en culture selon le procédé décrit dans Pain et col. (1996), incorporé par référence. Trente six heures plus tard les cultures ont été additionnées de G418 à la concentration de 250 µg/ml. Le milieu de culture contenant le G418 a ensuite été changé tous les jours pendant 4 jours, puis tous les deux jours. Des clones de cellules ES résistantes au G418 sont devenus apparents après le sixième jour. Ils furent prélevés individuellement entre 8 et 10 jours après le début de la culture. Ces clones furent ensemencés individuellement dans du milieu de culture frais contenant du G418 afin d'être amplifiés. Ils furent ensuite conservés dans de l'azote liquide.

Dans les cellules électroporées, l'expression du marqueur ROSA-β-geo a été analysée par l'identification *in situ* de l'activité β-galactosidase selon le procédé suivant. Les cellules en suspension furent fixées à 4°C pendant une durée de 30 minutes dans un mélange à base de PBS contenant 1 % de formaldéhyde, 0,2 % de glutaraldéhyde et 0,02 % de Nonidet P-40. Les cellules furent ensuite incubées à 37°C pendant une durée qui pouvait aller de 1 à 24 heures dans du PBS contenant 1mg/ml de 5-bromo-chloro-3-indolyl β-D-galactopuranoside, 5 mM de K₃Fe (CN)₆, 5 mM de K₄Fe(CN)₆, 2 mM de MgCl₂ et 0,02 % de Nonidet P-40. Les cellules exprimant le marqueur β-galactosidase étaient colorées en bleu.

L'objectif était d'identifier des cellules ES dans lesquelles le vecteur ROSA-β-geo serait inséré en aval d'un promoteur qui ne fonctionnerait que dans les cellules ES lorsque celles-ci sont pluripotentes. Après caractérisation de plusieurs clones, un clone a été retenu, appelé 9N2.5, qui ne présentait la réaction positive du test β-galactosidase que lorsque les cellules étaient maintenues dans les conditions de culture assurant la persistance du caractère pluripotent des cellules telles que décrites dans Pain et col. (1996). La positivité du test fut perdue lorsque les cellules 9N2.5 furent induites dans la différenciation (voir plus loin).

Le clone 9N2.5 a été amplifié en culture *in vitro* puis stocké sous forme viable par congélation dans l'azote liquide.

### Exemple 2 : Caractérisation des cellules 9N2.5.

Les cellules 9N2.5 ont été maintenues dans les conditions de culture décrites par Pain et col. (1996), pour les cellules ES de poulet. Dans ces conditions il a été vérifié que les cellules 9N2.5 présentaient la morphologie, l'activité télomérase et les épitopes antigéniques caractéristiques des cellules ES de poulet tels que décrits par Pain et col.. Ces cellules sont également capables de former des corps embryoïdes, comme les cellules parentales. L'électroporation, la sélection dans le G418 et l'amplification ultérieure de ces cellules n'avaient donc pas altéré leurs caractères de cellules ES.

Pour analyser l'expression du marqueur ROSA-β-geo dans les cellules différenciées, les cellules 9N2.5 furent induites dans la différenciation selon les procédés décrits dans Pain et al. Ces cellules furent cultivées en absence de cellules nourricières, en absence de LIF et de cytokines et en présence soit d'acide rétinoïque à la concentration de 5 x 10⁻⁶ M, soit de DMSO à la concentration de 1 %. Dans certaines cultures, l'acide rétinoïque et le DMSO furent ajoutés simultanément. Dans les milieux induisant la différenciation des cellules ES on a pu observer l'apparition de cellules différenciées identiques à celles qui furent décrites initialement par Pain et col. (1996) dans les mêmes conditions.

Après 4 jours de culture dans les milieux de différenciation, les cellules devinrent complètement négatives pour le test de l'activité β-galactosidase. Afin de confirmer l'absence d'expression du transgène ROSA-β-geo, son expression fut suivie soit par la recherche des ARNm LacZ par la technique de RT-PCR. Pour cela, les amorces SEQ ID N° 3 et SEQ ID N° 4 furent utilisées :

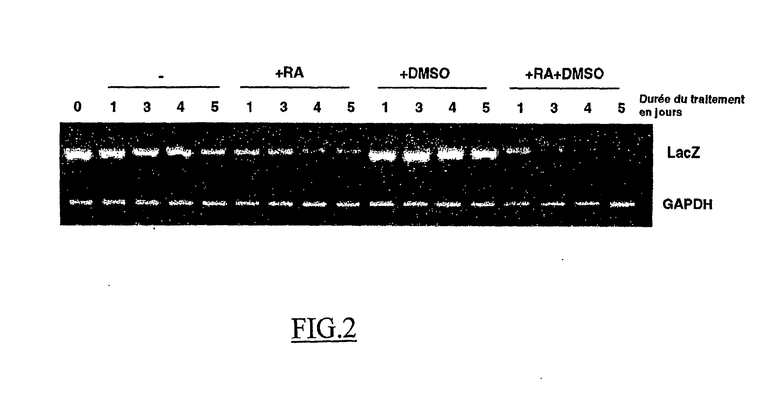

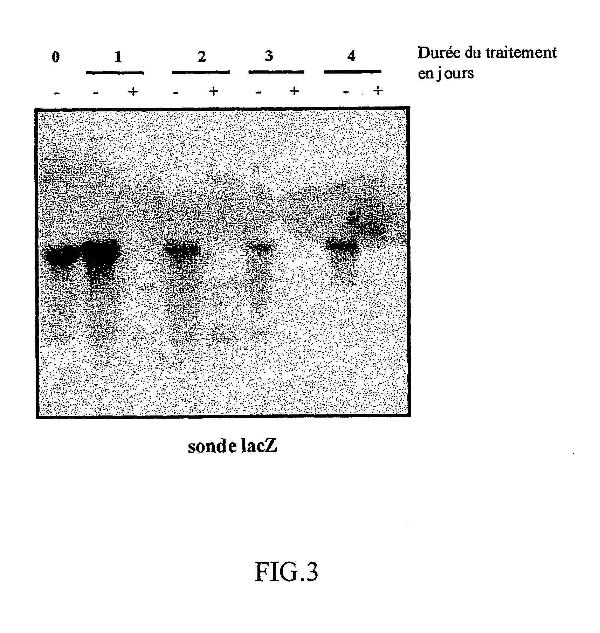

Comme il est montré sur la figure 2, la quantité d'ARN produite par le transgène ROSA-β-geo ne change pas durant 5 jours de culture des cellules dans le milieu de culture qui maintient la pluripotence (milieu ES). Par contre dans les milieux de culture de différenciation contenant soit l'acide rétinoïque seul, soit le DMSO, soit l'acide rétinoïque et le DMSO, la quantité d'ARNm ROSA-β-geo diminua très fortement après 4 jours de culture. Pour confirmation, les ARNm ROSA-β-geo furent aussi analysés par la technique de *Northern blot* en utilisant une sonde marquée spécifique de la séquence LacZ. Comme cela est montré dans la figure 3, en présence d'acide rétinoïque, les ARNm LacZ devinrent quasiment indétectables après deux jours de culture alors que leur expression était maintenue dans le milieu de culture dépourvu d'acide rétinoïque.

### Conclusion

Les cellules 9N2.5 expriment sélectivement le transgène ROSA-β-geo lorsqu'elles sont maintenues à l'état pluripotent. L'expression du transgène cesse très rapidement après l'induction de la différenciation de ces cellules en culture.

### Exemple 3 : Test de l'expression du transgène ROSA-β-geo dans les cellules 9N2.5 in vivo.

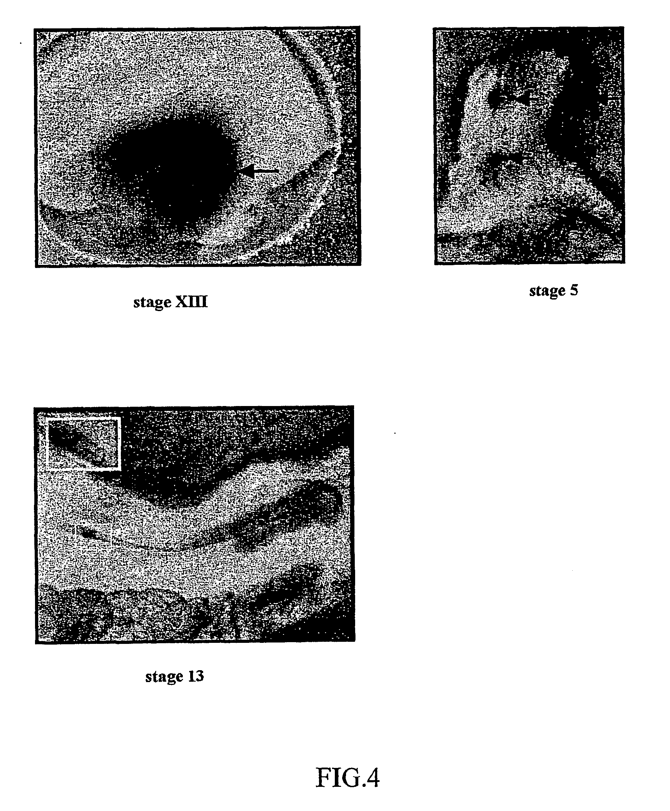

Afin d'analyser les potentialités de développement des cellules 9N2.5 et l'expression du transgène ROSA-β-geo dans un embryon *in vivo,* les cellules 9N2.5 ont été greffées dans des embryons de poulet au stade X selon l'échelle de Eyal-Giladi et Kochav (1976) (échelle E-G&K) selon le protocole décrit par Pain et col. (1996). La présence des descendants des cellules injectées a été recherchée dans les embryons à différents stades de développement après la greffe, par le test de la β-galactosidase. Comme cela est illustré sur la figure 4, des amas de cellules positives pour la β-galactosidase furent détectés dans l'épiblaste des embryons ayant atteint le stade XIII dans les embryons injectés. Ces cellules positives ne furent identifiées que dans l'épiblaste de l'aire pellucide. Plus tard au cours du développement, au stade de la gastrulation, stade 5 selon l'échelle de Hamburger et Hamilton (H&H), des cellules positives ne furent retrouvées que dans la ligne primitive et le croissant germinal extra-embryonnaire. Dans la ligne primitive, les cellules furent identifiées dans quelques amas pour la plupart localisés dans le noeud de Hensen. Au stade 13 (échelle H&H), des cellules positives ne furent trouvées que dans le sinus rhomboidal qui correspond à la plaque neurale encore ouverte dans la partie caudale de l'embryon. Plus tard au cours du développement embryonnaire, des cellules positives n'ont été retrouvées que sous forme de très rares cellules isolées dans certains tissus d'origine nerveuse, ainsi que dans les ébauches gonadiques.

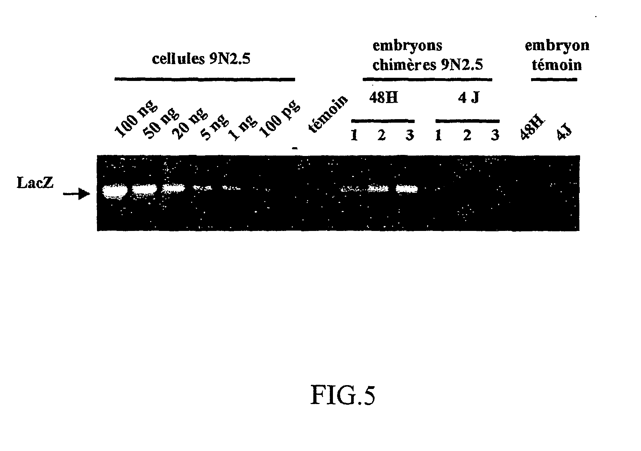

Afin de vérifier si, malgré la négativité de la réaction β-galactosidase, des descendants des cellules 9N2.5 avaient bien colonisé en nombre les tissus d'embryons âgés, la présence du transgène ROSA-β-geo a été recherchée par PCR dans de l'ADN extrait à partir d'embryon complet âgé de 2 ou 4 jours de développement. Comme cela est montré sur la figure 5, une bande caractéristique du transgène ROSA-β-geo a pu être détectée, démontrant que les cellules descendantes des cellules 9N2.5 greffées étaient présentes au moins 4 jours après la greffe.

Quelques embryons injectés avec des cellules 9N2.5 ont achevé leur développement et ont donné naissance à des poussins. La recherche des séquences du transgène ROSA-β-geo a été entreprise sur l'ADN isolé de divers tissus par la technique de PCR. Ainsi chez deux poussins qui ont été analysés on a révélé la présence du transgène dans la peau, le gésier, le foie. Tous ces tissus ne présentaient pas d'activité β-galactosidase ce qui démontre que les transgènes étaient présents dans des cellules différenciées issues des cellules 9N2.5 greffées.

### Conclusion

Les cellules 9N2.5 sont donc capables de coloniser un embryon hôte et de s'y développer. Cependant l'expression du transgène ROSA-β-geo reste limitée aux cellules très tôt après la transplantation dans l'embryon ainsi qu'à de rares cellules présentent dans quelques tissus comme les gonades ou le système nerveux. Compte tenu des observations effectuées sur les cellules 9N2.5 en culture, on peut raisonnablement imaginer que l'expression du transgène ROSA-β-geo dans les cellules *in vivo* se limite aux cellules qui ne se sont pas encore engagées dans la différenciation.

L'ensemble de ces données obtenues *in vitro* et *in vivo* à partir des cellules 9N2.5 conduit à supposer que le transgène ROSA-β-geo est inséré dans un locus du génome de ces cellules dont l'activité transcriptionnelle est spécifique des cellules ES à l'état pluripotent.

### Exemple 4: Prolifération des cellules 9N2-5 in vivo.

Afin d'analyser si les cellules 9N2.5 étaient capables de proliférer dans certains compartiments de l'embryon, deux embryons injectés ont été prélevés après 7 jours d'incubation. Les embryons ont été arbitrairement coupés en 3 parties : la tête, le tronc en incluant les ébauches des membres supérieurs, la queue en incluant les ébauches des membres inférieurs. Ces parties ont été dissociées dans la pronase et la suspension cellulaire ensemencée en culture selon le procédé de culture décrit par Pain et col. ( 1996). Une sélection au G418 à 250 µg/ml fut réalisée pendant 6 jours. Il apparut quelques foyers de cellules résistantes dans toutes les cultures, mais la fréquence de ces foyers étaient beaucoup plus élevée dans les cultures ensemencées à partir de la partie postérieure des embryons. Les cellules résistantes au G418, issues de cette culture furent repiquées pour être amplifiées, 7 jours après l'ensemencement initial. Une partie de ces cultures parallèles fut testée positivement pour l'expression de l'activité β-galactosidase. Cette approche a permis, pour un des 2 embryons testés, de maintenir, d'amplifier et même de congeler, sous forme viable, des cellules positives pour la β-galactosidase, résistantes au G418 et qui présentaient une morphologie identique à celle des cellules 9N2.5 injectées. Les cellules issues du deuxième embryon, bien que positives pour l'activité β-galactosidase, n'ont proliféré que lentement et n'ont pas pu être suffisamment amplifiées.

### Conclusion

Ces résultats montrent donc que certaines cellules 9N2.5 sont capables de se maintenir sous forme de cellules ES dans certaines régions de l'embryon. Ces cellules correspondent vraisemblablement aux rares cellules positives pour la β-galactosidase identifiées sur les coupes d'embryons injectés avec les cellules 9N2.5 (voir ci-dessus). Au regard de leur localisation dans la partie postérieure de l'embryon, on peut suggérer que certaines des cellules qui conservent les caractères des cellules 9N2.5 *in vivo* correspondent à des cellules EG telles que décrites chez la souris et chez l'homme (Matsui et al. 1992, Shamblott et al. 1998). Les cellules EG sont des cellules précurseurs des cellules germinales qui possèdent des propriétés de pluripotence et des caractères cytologiques très voisins de ceux des cellules ES.

### Exemple 5 : Utilisation des cellules 9N2.5 pour un criblage de substances

Les cellules 9N2.5 présentent une expression de la β-galactosidase forte quand elles sont dans un état indifférencié. Cette expression est perdue lors d'une induction de différenciation. Cette propriété peut être mise à profit pour tester différentes molécules inductrices ou promotrices de la différenciation ou pour tester des molécules non inductrices. Les cellules 9N2.5 peuvent ainsi être utilisées comme support de test pour identifier des lots de sérum appropriés à la culture de cellules ES ou bien à leur différenciation. Pour cela, les cellules sont ensemencées dans un milieu identique à celui employé pour maintenir les cellules parentales. Dans ce milieu, le sérum de référence est remplacé par les différents sera à tester, à différentes concentrations éventuellement. Les ensemencements sont réalisés à très faible densité (2 x 10⁴ cellules par boîte de 35 mm) et les cellules cultivées pendant 4 jours. Les cellules sont alors fixées, colorées pour révéler l'activité β-galactosidase et le nombre de foyers positifs estimé. Le nombre de foyers positifs est en relation directe avec la capacité du sérum à maintenir l'autorenouvellement des cellules ES. Cet exemple peut être étendu au test de substances diverses, naturelles ou de synthèse.

### Conclusion

Les cellules 9N2.5 peuvent être utilisées pour cribler des substances sur leur aptitude à induire l'autorenouvellement ou bien la différenciation de cellules ES en culture.

### Exemple 6 : Identification du locus d'intégration du transgène ROSA-β-geo dans les cellules ES 9N2.5

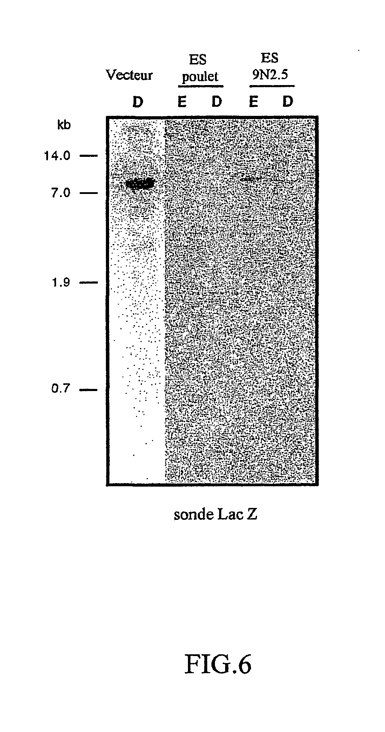

Dans une première approche vers l'identification du locus d'intégration du transgène ROSA-β-geo dans les cellules 9N2.5 une analyse de l'ADN génomique des cellules 9N2.5 par la technique de *Southern Blot* a été effectuée. L'ADN de cellules 9N2.5 fut digéré par l'enzyme de restriction *Eco*RI ou par l'enzyme *Dra*I qui ne coupent chacune le transgène ROSA-β-geo qu'en un site unique. Après migration électrophorétique de l'ADN digéré, les filtres furent hybridés avec une sonde spécifique du fragment LacZ. Comme cela est montré sur la figure 6, une seule bande a été identifiée dans ces conditions dans chacune des digestions pratiquées. Aucune bande ne fut identifiée dans l'ADN de cellules ES normales de poulet ne contenant pas le transgène ROSA-β-geo. Ces résultats démontraient que, dans les cellules 9N2.5, une seule copie du transgène ROSA-β-geo est intégrée.

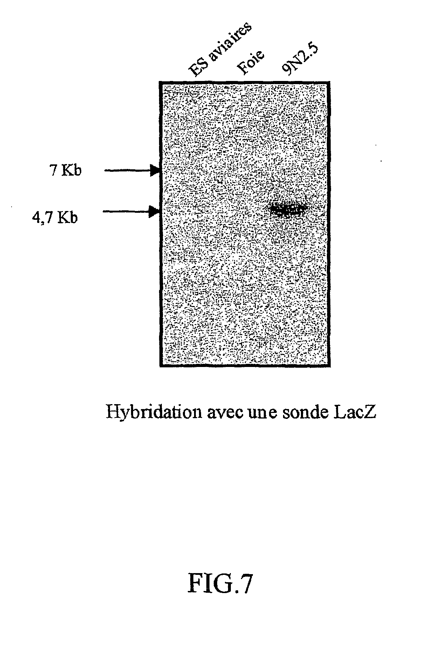

Dans un second temps, la taille de l'ARNm transcrit à partir du transgène a été analysé. De l'ARN de cellules 9N2.5 a été analysé par *Northern blot* avec une sonde LacZ. Comme cela est montré sur la figure 7, un seul transcrit de taille 4,7 kb a été révélé. Ce transcrit n'est pas présent dans l'ARN de cellules ES normales. Compte tenu de la longueur attendue de la séquence qui doit être transcrite dans le transgène ROSA-β-geo, soit 3,9 kb, on doit imaginer que le transcrit révélé dans les cellules 9N2.5 contient environ 0,8 kb de séquences issues du gène cellulaire dans lequel est inséré le transgène. Ces séquences cellulaires peuvent se situer sur l'ARNm soit en 5', soit en 3', soit être réparties des deux côtés de la séquence transcrite du transgène ROSA-β-geo. Afin de les rechercher en région 5', la technique 5'-RACE en utilisant le kit *Marathon* de la société Clontech a été mise en oeuvre.

À partir d'ARN de cellules 9N2.5, un brin d'ADN complémentaire fut synthétisé en utilisant une amorce spécifique de la région LacZ, amorce de séquence (SEQ ID N° 5).

Après synthèse du second brin complémentaire de ce premier brin, l'ADN complémentaire double brin fut lié à l'adaptateur fourni dans le kit *Marathon* dont la séquence est SEQ ID N° 6. L'ensemble de la séquence fusionnée fut ensuite amplifié par la technique de PCR en utilisant les amorces SEQ ID N° 7 et SEQ ID N° 8.

L'amplification fut réalisée sur une machine *2400 Perkin Elmer* dans les conditions suivantes: 94°C pendant 30 secondes, puis 5 cycles à 94°C de 5 secondes chacun, puis 4 minutes à 72°C, puis 5 cycles à 94°C de 5 secondes chacun, puis 4 minutes à 70°C, puis 25 cycles à 94°C de 5 secondes chacun, puis 4 minutes à 68°C. Un produit d'amplification de 400 paires de bases fut identifié. Ce fragment appelé F1 fut cloné dans un plasmide pour être amplifié, puis sa séquence exacte fut déterminée. Nous avons ensuite recherché des séquences situées en aval de la séquence F1 sur l'ARNm transcrit dans les cellules ES en utilisant la technique de RT-PCR. Pour cela de l'ARN de cellules ES normales fut utilisé comme matrice pour synthétiser un ADN complémentaire par amorçage au moyen d'une amorce P3 de séquence SEQ ID N° 9.

L'ADN complémentaire monocaténaire fut ensuite amplifié par PCR au moyen des amorces SEQ ID N° 10 qui correspond à la séquence 5' du fragment initialement amplifié par la technique 5'-RACE, et SEQ ID N° 11.

Un fragment appelé C1 fut ainsi amplifié puis cloné dans un plasmide. La séquence exacte de C1 fut déterminée (SEQ ID N° 12).

Afin de confirmer que la séquence C1 est bien dans les ARNm qui portent aussi la séquence LacZ dans les cellules 9N2.5, une amplification par RT-PCR a été effectuée sur les ARNm issus de ces cellules en utilisant les amorces respectives P4 (SEQ ID N° 13), spécifique du fragment C1, et LacZB (SEQ ID N°8), spécifique de la séquence LacZ.

Un fragment de 331 paires de bases fut identifié. La taille de ce fragment correspond à celle attendue ce qui indique que la séquence C1 et la séquence LacZ se trouvent bien sur un même ARNm. La confirmation était ainsi apportée que la séquence C1 doit être spécifique du gène cellulaire dans lequel est inséré le transgène ROSA-β-geo. Ce gène a été appelé *ens-1* (embryonic normal stem cell gene).

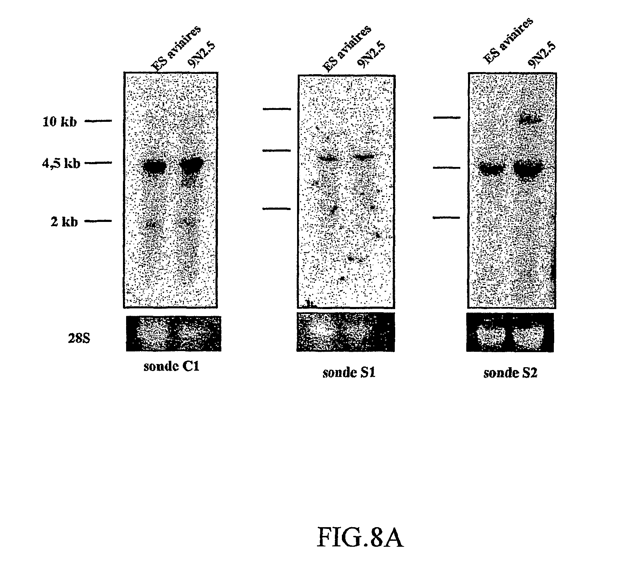

Afin de vérifier que le gène *ens-1* produit bien un ARN messager les ARN de cellules ES normales de poulet ont été analysés par la technique de *Northern blot* au moyen de la sonde C1. Comme cela est montré sur la figure 8A, la sonde C1 identifie un ARN majeur de taille voisine de 4,7 kb ainsi que deux ARN très faiblement marqués d'environ 10 kb et 2 kb respectivement.

À partir de la séquence C1, le clonage de l'ARNm complet transcrit à partir du gène *ens-1* a été entrepris.

Pour cela une banque d'ADNc construite à partir d'ARN polyadénylés isolés de cellules ES de poulet a été criblée avec des sondes préparées à partir du fragment C 1.

Un ADN complémentaire de 4,2 kpb a été isolé. Afin de vérifier si cet ADNc est bien représentatif de l'ARNm transcrit à partir du gène *ens-1,* deux sondes nucléotidiques ont été préparées, respectivement S1 et S2, correspondant à deux fragments différents de cet ADNc situés en aval de la séquence C1. Ces deux sondes ont été utilisées pour identifier, par la technique de *Northern blot,* les ARN correspondants isolés de cellules ES normales de poulet. Comme cela est montré sur la figure 8A, ces deux sondes identifient un ARN de taille voisine de 4,5 kb, identique à celle de l'ARN majoritaire identifié précédemment avec la sonde C1. Comme cela est montré plus loin le patron d'expression de cet ARN identifié avec les deux sondes S1 et S2 est identique à celui de l'ARN majoritaire identifié avec la sonde C1 dans les cellules ES normales.

L'ensemble de ces données suggère très fortement que les sondes C1, S1 et S2 reconnaissent le même ARNm *ens-1* dans les cellules ES normales de poulet.

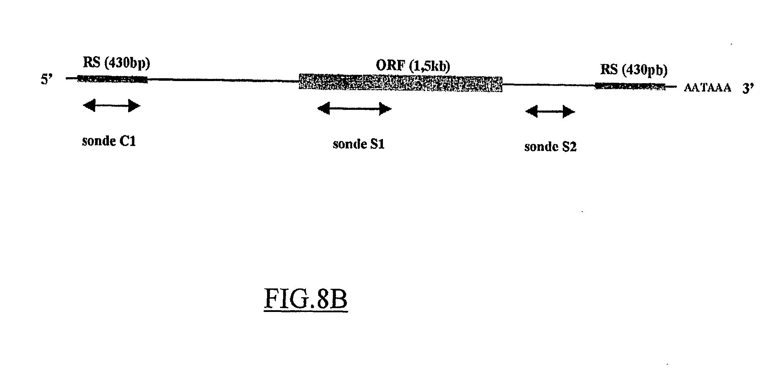

La séquence de l'ARNm *ens-1* est présentée dans SEQ ID N° 1, et la structure de l'ADNc est présentée dans la figure 8B. L'analyse de cette séquence révèle un cadre de lecture de grande longueur pouvant coder pour une protéine de 490 acides aminés dont la séquence est présentée par SEQ ID N° 2. Il est à noter que la séquence C1 est polymorphique et que celle obtenu à partir du clone d'ADNc, et présentée dans SEQ ID N° 1, est légèrement différente de celle obtenue précédemment par 5'RACE (SEQ ID N° 12).

Afin de vérifier si le gène *ens-1* correspond bien au gène dans lequel est inséré le transgène ROSA-β-geo dans les cellules 9N2.5 le patron d'expression du gène *ens-1* au cours du développement embryonnaire du poulet et au cours de la différenciation des cellules ES de poulet en culture a été analysé en utilisant la technique de *Northern blot.*

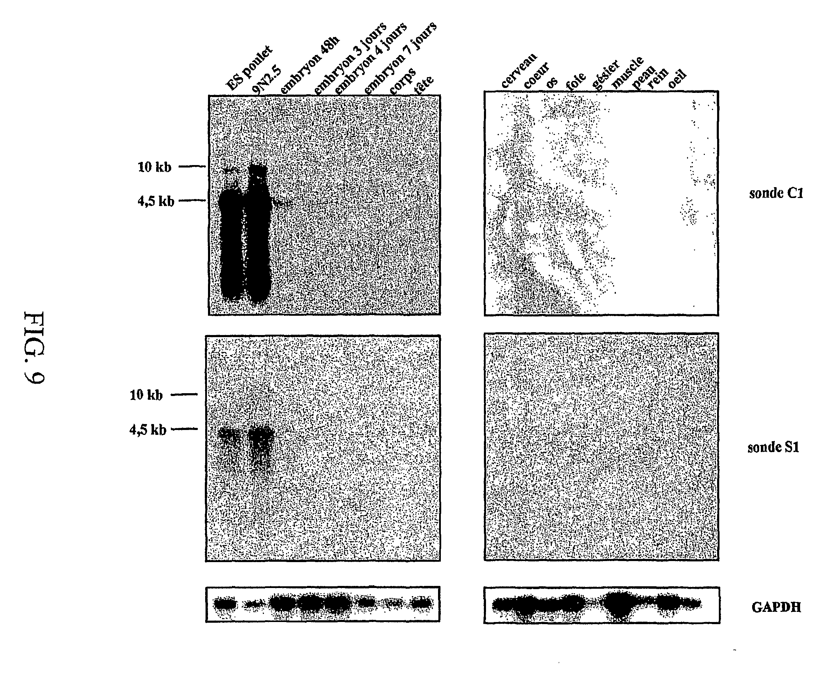

Comme cela est montré dans la figure 9, la sonde C1 et la sonde S1 identifient le même ARN de 4,5 kb dans les ARN extraits d'embryon de poulet normal de 48 heures. L'intensité du signal diminue fortement dans les ARN extraits d'embryons plus âgés, comme les embryons de 3 jours et de 4 jours. Le signal disparaît dans les ARN extraits d'embryons âgés de 7 jours ou de 18 jours. Il est nul dans les ARN extraits de divers tissus de poussin comme le foie, le muscle, le gésier, le cerveau, le coeur, l'oeil, l'os ou la peau.

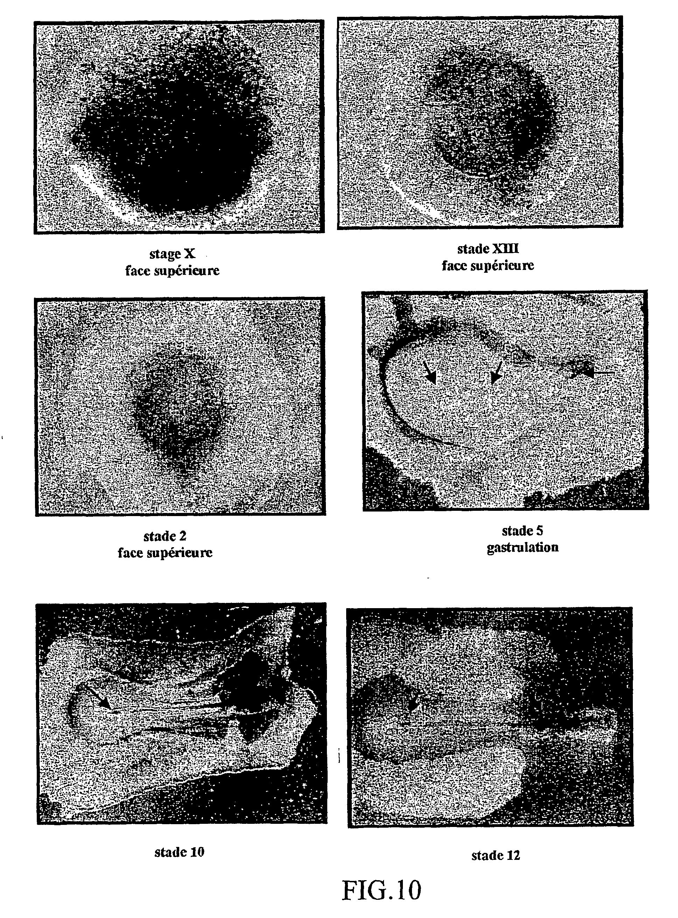

Afin de déterminer plus précisément le patron d'expression du gène *ens-1* durant les premiers stades du développement de l'embryon de poulet, les ARNm *ens-1* ont été recherchés par la technique d'hybridation *in situ* sur embryon total. Les résultats sont présentés sur la figure 10. Un signal très fort fut observé dans l'aire pellucide d'embryon aux stade X et XIII (échelle E-G&K). Dans les embryons au stade 2 (échelle H&H), le signal ne fut trouvé que dans l'aire pellucide avec une forte dominance dans la région de la ligne primitive. Au stade 5 (échelle H&H) le signal fut retrouvé dans le noeud de Hensen et dans la région rostro-caudale de la ligne primitive, ainsi que sous une forme très prononcée dans le croissant germinal en position antérieure de l'embryon. À des stades plus avancés du développement embryonnaire, aucun signal significatif ne fut détecté. Les mêmes patrons d'expression furent observés avec les sondes C1 et S1.

### Conclusion

Le gène *ens-1* présente une expression spécifique des cellules ES de poulet indifférenciées et des stades très précoces de l'embryogenèse. L'expression du gène devient très faible voire indétectable après l'achèvement de la gastrulation.

Le gène *ens-1* constitue donc un marqueur très spécifique des cellules embryonnaires indifférenciées, que ces cellules soient présentes dans l'embryon, ou qu'elles soient maintenues dans cet état en culture *in vitro.* Le gène *ens-1* est aussi spécifique des cellules du croissant germinal et donc des cellules précurseurs des gamètes.

### Exemple 7 : Conservation du gène ens-1 au cours de l'évolution

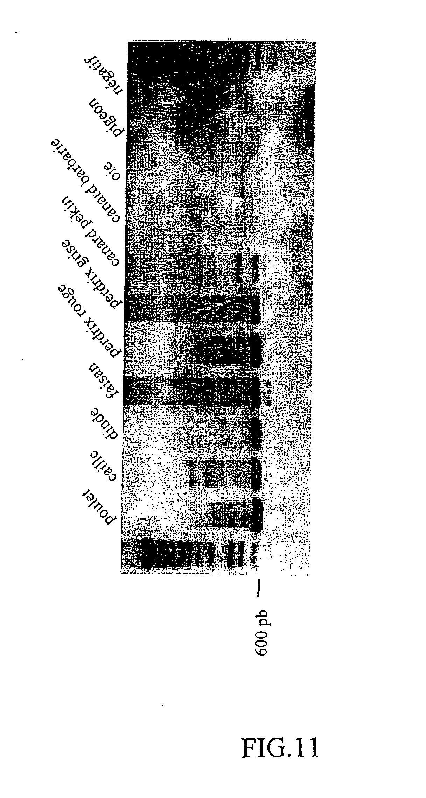

Afin d'analyser le degré de conservation du gène *ens-1* au cours de l'évolution une sonde spécifique du gène *ens-1* de poulet a été utilisée pour hybrider de l'ADN génomique provenant de diverses especes animales par la technique du southern blot (non montré). La technique d'amplification de séquence nucléique par PCR entre deux amorces spécifiques du gène *ens-1* (SEQ ID N° 14 et SEQ ID N° 15), en utilisant le protocole : 96°C 3 minutes, (96°C 30 s, 62°C 30s, 72°C 30s, 10 cycles), (96°C 30s, 57°C 30s, 72°C 30s, 10 cycles), (96°C 30s, 52°C 30s, 72°C 30s, 20 cycles), a également été utilisée. Les résultats présentés sur la figure 11 montrent que des séquences homologues sont retrouvées uniquement dans l'ordre des galliformes (poulet, caille, dinde, faisan, perdrix rouge, perdrix grise). Il est à noter qu'aucune homologue à *ens-1* n'est trouvé chez les mammifères (non montré).

### Exemple 8: Identification dans le gène ens-1 d'une séquence promoteur de transcription dont l'activité est spécifique des cellules souches embryonnaires.

Le gène *ens-1* a donc été identifié comme étant un gène spécifiquement exprimé dans les cellules souches embryonnaires de poulet.

Une région promoteur dont l'activité transcriptionnelle est spécifique des cellules ES indifférenciées de poulet a été identifiée dans le gène *ens-1.* Les applications sont importantes car cela permet de disposer ainsi d'un outil génétique qui permettrait de cibler l'expression d'un transgène spécifiquement dans les cellules souches embryonnaires et vraisemblablement aussi dans les embryons de poulet au stade précédent la gastrulation.

La présence de séquences répétées aux extrémités du transcrit *ens-1* suggérait que ces séquences soient apparentées aux séquences LTR (long terminal repeat) des rétrovirus. Les LTR de rétrovirus sont régionalisées en trois parties, respectivement, U3, R et U5 (dans le sens 5'-3'). Dans le génome rétroviral, la région U3 est capable d'activer la transcription avec parfois un contrôle tissu-spécifique. Dans les ARN messagers rétroviraux, une copie des séquences R-U5 se trouve en 5' et une copie des séquences U3-R se trouve en 3'.

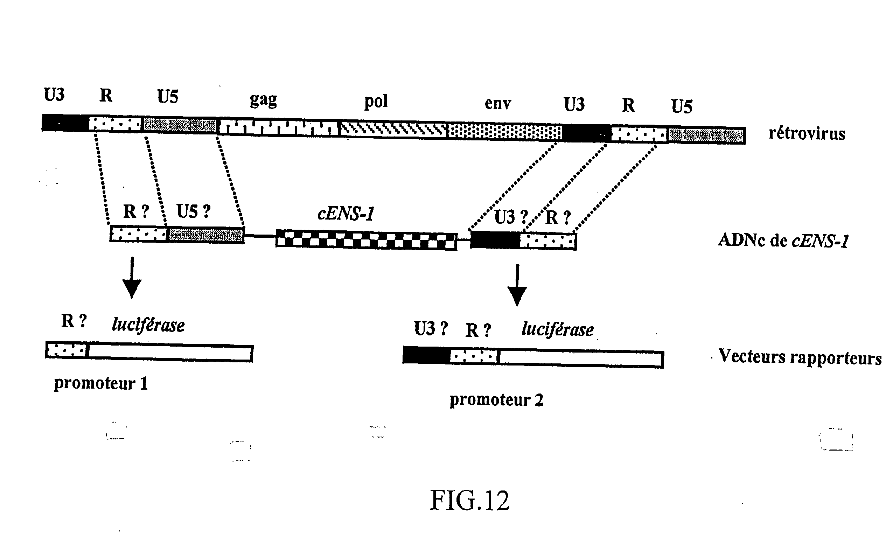

Par analogie avec la structure des LTR de rétrovirus, les régions pouvant correspondre aux régions U3, R et U5 des rétrovirus ont été identifiées dans l'ARN messager du gène *ens-1.* La séquence identifiée comme étant répétée aux deux extrémités du transcrit *ens-1* correspond à la région R et la séquence qui correspondrait à la région U3 se localise entre l'extrémité 3' de la séquence codante pour ens-1 et l'extrémité 5' de R. (fig 12).

Pour tester l'activité promotrice des régions R et U3-R du gène *ens-1,* ces régions ont été clonées dans les deux orientations possibles, sens et antisens, en amont du gène rapporteur de la luciférase de luciole pour obtenir respectivement les vecteurs appelés promoteur 1 et promoteur 2, respectivement sens (S) et anti-sens (AS). (Fig 12). Ces constructions ont été transfectées dans différentes lignées cellulaires, dont des cellules souches de poulet 9N2-5, avec pour contrôle interne d'efficacité de transfection, le vecteur pRL-CMV (Promega) contenant le gène de luciferase de la chenille Renilla sous le contrôle du promoteur du cytomegalovirus.

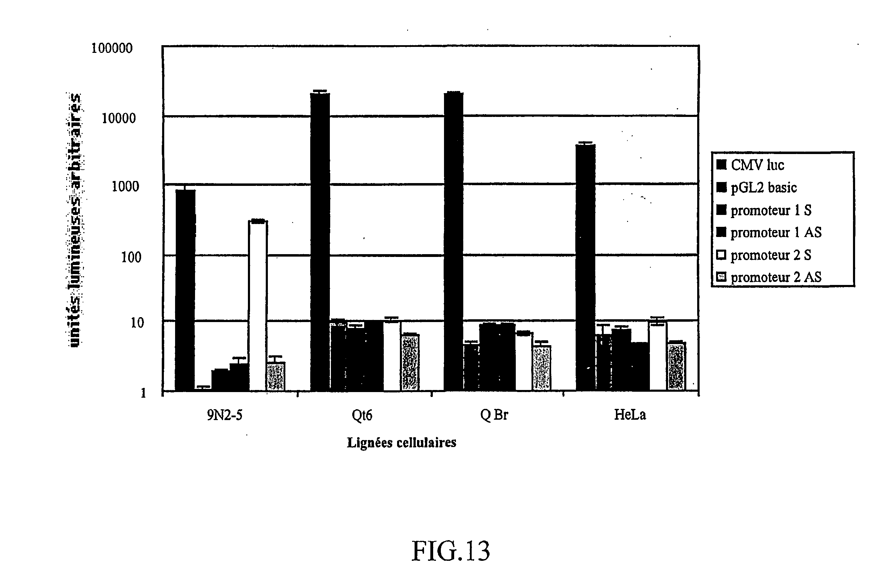

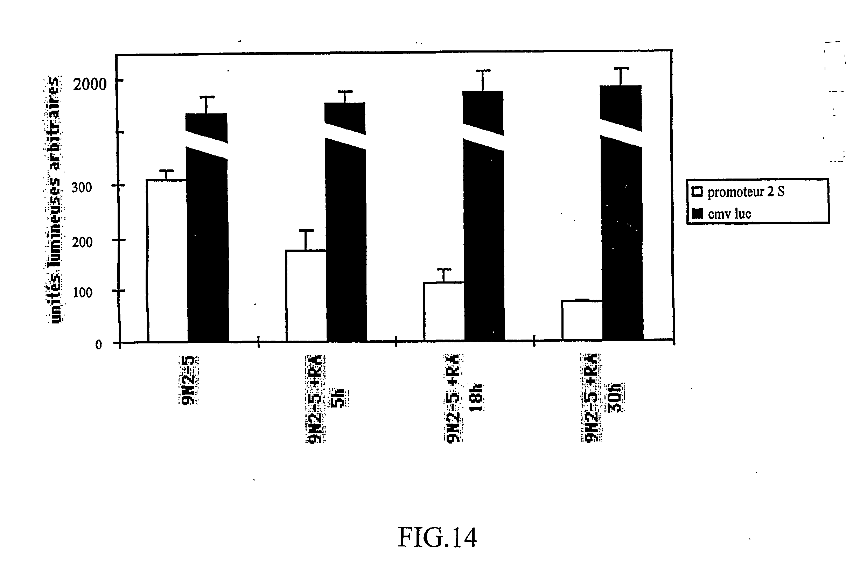

La transfection des divers vecteurs promoteur 1 et promoteur 2 dans les cellules 9N2-5 et la mesure de l'activité luciférase normalisée grâce au contrôle interne ont permis d'identifier une activité transcriptionnelle de la région U3 du vecteur promoteur 2S dans les cellules souches embryonnaires de poulet (cellules 9N2-5) alors que la région R ne montre aucune activité significative (Fig 13). L'activité du promoteur est par contre très faible dans les différentes autres lignées cellulaires testées (fibroblastes de caille Qt6, cellules épithéliales de caille QBr ou cellules épithéliales humaines). D'autre part le vecteur promoteur 2S a été transfecté dans des cellules souches embryonnaires 9N2-5 induites à se différencier par traitement à l'acide rétinoïque. La mesure de l'activité luciférase dans les cellules à différents temps après le traitement par l'acide rétinoïque montre que l'activité transcriptionnelle du promoteur décroît au cours de la différenciation des cellules souches embryonnaires alors que l'activité du promoteur contrôle (CMV) reste forte. (Fig 14).

L'ensemble de ces résultats montre qu'il existe, en 3' de la séquence codante du gène ens1, une région dotée d'une activité promotrice de la transcription et que cette activité transcriptionnelle est spécifique des cellules souches embryonnaires de poulet indifférenciées.

En utilisant la technique 5' RACE sur le vecteur promoteur 2S il a été possible de déterminer un site initiateur de transcription sur la séquence de l'ADNc *ens-1* (SEQ ID N° 1), ainsi qu'une séquence de type promoteur TATA an amont de ce site initiateur de transcription. Le promoteur correspond aux nucléotides 3111-3670 de SEQ ID N° 1.

### DEPOT DE MATERIEL BIOLOGIQUE

La lignée cellulaire 9N2.5 a été déposée le 11 Mai 2000 à la Collection Nationale de Cultures de Microorganismes (CNCM), 25 rue du Docteur Roux, 75724 Paris Cedex 15, France, selon les dispositions du Traité de Budapest, sous le numéro d'ordre I-2477, et correspond à la lignée de cellules souches embryonnaires de poulet dans laquelle le transgène ROSA-β-geo linéarisé par *Dra*I a été introduit par électroporation, et qui ont été isolées après sélection au G418, et pour leur activité β-galactosidase, ainsi que décrit dans l'exemple 1.

Les cellules utilisables pour cultiver les cellules 9N2.5 (fibroblastes de souris, STO) ont également été déposées à la CNCM le 11 Mai 2000, sous le numéro SH-2477.

### Références

Buckholz, (1993), Curr. Op. Biotechnology 4, 538.
Carter, (1993) Curr. Op. Biotechnology 3, 533.
Duck et al. (1990), Biotechniques, 9, 142.
Edwards et Aruffo (1993), Curr. Op. Biotechnology, 4, 558.
Epstein (1992) Médecine/Sciences, 8, 902.
Etches et al. (1996). Science 76, 1075-1083.
Eyal-Giladi et Kovak (1976). Dev. Biol. 151, 75-585.
Freidrich et Soriano (1991). Genes & Development 5, 1513-1523.
Guatelli et al. (1990), Proc. Natl. Acad. Sci. USA 87: 1874.
Kemler et al. (1981). J. Embryol. Exp. Morph. 64, 45-60.
Kievitis et al. (1991), J. Virol. Methods, 35, 273.
Köhler et Milstein. (1975) Nature 256, 495.
Kwoh, et al. (1989), Proc. Natl. Acad. Sci. USA, 86, 1173.
Landegren et al. (1988) Science 241, 1077.
Luckow (1993), Curr. Op. Biotechnology 4, 564.
Matsui et al. (1992). Cell 70, 841-847.
Matthews et al. (1988), Anal. Biochem., 169, 1-25.
Miele et al. (1983), J. Mol. Biol., 171, 281.
Neddleman et Wunsch (1970) J. Mol. Biol. 48 : 443
Olins et Lee (1993), Curr. Op. Biotechnology 4 : 520.
Pain et al. (1996). Development 122, 2339-2348.
Pain et al. (1999). Cells Tissues Organes 165, 212-219.
Perricaudet et al. (1992). La Recherche 23 : 471.
Pearson et Lipman (1988) Proc. Natl. Acad. Sci. USA 85 : 2444
Prowse et Greider (1995). Proc Natl Acad Sci USA 92, 4818-4822.
Rohlmann et al. (1996) Nature Biotech. 14 : 1562.
Rolfs, A. et al. (1991), Berlin : Springer-Verlag.
Rosner et al. (1990). Nature 354, 686-692.
Sambrook et al. (1989) Molecular cloning : a laboratory manual. 2nd Ed. Cold Spring Harbor Lab., Cold Spring Harbor, New York.
Segev, (1992), Kessler C. Springer Verlag, Berlin, New-York, 197-205.
Shamblott et al. (1998). Proc Natl Acad Sci USA 95, 13726-13731.
Smith et Waterman (1981) Ad. App. Math. 2 : 482
Solter et Knowles (1978) Proc Natl Acad Sci USA. 75, 5565-5569.
Stewart et Yound (1984), Solid phase peptides synthesis, Pierce Chem. Company, Rockford, 111, 2ème éd., (1984).
Strickland et al. (1980). Cell 21, 347-355.
Temin, (1986) Retrovirus vectors for gene transfer. In Kucherlapati R., ed. Gene Transfer, New York, Plenum Press, 149-187.
Walker (1992), Nucleic Acids Res. 20 : 1691.

## Revendications

1. Acide nucléique purifié ou isolé, **caractérisé en ce qu'**il comprend une séquence nucléique choisie dans le groupe de séquences suivantes :
a) SEQ ID N°1, ou le fragment nucléotidique 1409-2878 de SEQ ID N°1;
b) le fragment nucléotidique 3111-3670 de SEQ ID N°1 ;
c) une séquence nucléique présentant un pourcentage d'identité d'au moins 80 %, après alignement optimal avec une séquence définie en a), ou b), possédant un rôle dans le caractère de pluripotence des cellules ES et utilisable comme marqueur du caractère de pluripotence des cellules ES ;
d) la séquence complémentaire ou la séquence d'ARN issue de la séquence telle que définie en a), b), ou c).

2. Acide nucléique purifié ou isolé selon la revendication 1, **caractérisé en ce qu'**il comprend ou est constitué de SEQ ID N°1, la séquence complémentaire ou la séquence d'ARN issue de l'une de ces séquences.

3. Acide nucléique purifié ou isolé **caractérisé en ce qu'**il code pour un polypeptide possédant un fragment continu d'au moins 200 acides aminés de la protéine SEQ ID N°2.

4. Polypeptide isolé **caractérisé en ce qu'**il comprend un polypeptide choisi parmi :
a) un polypeptide de séquence SEQ ID N°2,
b) un polypeptide comportant au moins 80 % d'identité avec ledit polypeptide de a), possédant un rôle dans le caractère de pluripotence des cellules ES et utilisable comme marqueur du caractère de pluripotence des cellules ES.

5. Polypeptide selon la revendication 4, **caractérisé en ce qu'**il est constitué d'une séquence choisie parmi SEQ ID N°2, ou une séquence possédant au moins 80 % d'identité avec cette séquence après alignement optimal.

6. Vecteur de clonage et/ou d'expression comprenant un acide nucléique selon l'une des revendications 1 à 3 ou codant pour un polypeptide selon l'une des revendications 4 et 5.

7. Cellule hôte à l'exception des cellules ES humaines, **caractérisée en ce qu'**elle est transformée par un vecteur selon la revendication 6.

8. Cellule hôte contenant un acide nucléique selon l'une des revendications 1 à 3, **caractérisée en ce qu'**il s'agit d'une cellule ES d'oiseau modifiée en outre par introduction d'un gène exogène, ledit gène exogène étant intégré dans ledit acide nucléique selon l'une des revendications 1 à 3 et étant uniquement et spécifiquement exprimé lorsque ladite cellule est maintenue à l'état pluripotent.

9. Cellule selon la revendication 8, **caractérisée en ce que** ledit gène exogène est un gène rapporteur.

10. Cellule selon la revendication 9, **caractérisée en ce que** ledit gène rapporteur est choisi parmi lacZ, GFP, luciférase, ROSA-β-geo, un gène de résistance à un antibiotique.

11. Cellule hôte contenant un acide nucléique selon l'une des revendications 1 à 3, **caractérisée en ce qu'**il s'agit d'une cellule d'oiseau modifiée en outre par introduction d'un acide nucléique exogène, ledit acide nucléique exogène étant intégré dans ledit acide nucléique selon l'une des revendications 1 à 3.

12. Cellule selon la revendication 11, **caractérisée en ce que** ledit acide nucléique exogène est un gène d'intérêt thérapeutique, éventuellement précédé d'un promoteur spatio-temporel et/ou de séquences terminatrices.

13. Cellule selon la revendication 11, **caractérisée en ce que** ledit acide nucléique exogène est un marqueur génétique.

14. Cellule selon l'une des revendications 8 à 13, **caractérisée en ce que** ledit oiseau appartient à l'ordre des Galliformes.

15. Cellule selon la revendication 14, **caractérisée en ce que** ledit oiseau est un poulet ou une caille.

16. Cellule selon l'une des revendications 14 et 15, **caractérisée en ce que** ledit gène rapporteur est intégré sous le contrôle du promoteur du gène *ens-*1 de séquence nucléotidique 3111-3670 de SEQ ID N°1.

17. Cellule selon l'une des revendications 14 et 15, **caractérisée en ce qu'**il s'agit d'une cellule 9N2.5, déposée à la Collection Nationale des Microorganismes le 11 Mai 2000 sous le numéro d'ordre 1-2477.

18. Cellule d'oiseau différentiée, **caractérisée en ce qu'**elle dérive d'une cellule ES selon l'une des revendications 8 à 17.

19. Animal excepté l'homme, **caractérisé en ce qu'**il comprend une cellule selon l'une des revendications 7 à 18.

20. Utilisation d'une séquence d'acide nucléique choisie parmi une séquence d'acide nucléique selon l'une des revendications 1 à 3, un fragment d'au moins 15 nucléotides consécutifs de la séquence SEQ ID N°1 et une séquence nucléique s'hybridant dans des conditions de forte stringence avec l'une de ces séquences, en tant que sonde ou amorce, pour la détection et/ou l'amplification de séquences d'acide nucléique selon l'une des revendications 1 à 3.

21. Utilisation d'une séquence d'acide nucléique choisie parmi un acide nucléique selon l'une des revendications 1 à 3, un fragment d'au moins 15 nucléotides consécutifs de la séquence SEQ ID N°1 et une séquence nucléique s'hybridant dans des conditions de forte stringence avec l'une de ces séquences, comme oligonucléotide sens ou antisens spécifique d'une séquence d'acide nucléique selon l'une des revendications 1 à 3.

22. Utilisation d'une séquence d'acide nucléique selon l'une des revendications 1 à 3 pour la production d'un polypeptide recombinant selon la revendication 4 ou 5.

23. Procédé d'obtention d'un polypeptide recombinant **caractérisé en ce que** l'on cultive une cellule selon la revendication 7 dans des conditions permettant l'expression dudit polypeptide et que l'on récupère ledit polypeptide recombinant, ledit polypeptide étant le polypeptide selon la revendication 4 ou 5.

24. Polypeptide recombinant **caractérisé en ce qu'**il est obtenu par un procédé selon la revendication 23.

25. Anticorps monoclonal ou polyclonal **caractérisé en ce qu'**il lie sélectivement un polypeptide selon l'une des revendications 4, 5 ou 24.

26. Procédé de détection d'un polypeptide selon l'une des revendications 4, 5 ou 24, **caractérisé en ce qu'**il comprend les étapes suivantes :
a) mise en contact d'un échantillon biologique avec un anticorps selon la revendication 25 ;
b) mise en évidence du complexe antigène-anticorps formé.

27. Trousse de réactifs pour la mise en oeuvre d'un procédé selon la revendication 26, **caractérisée en ce qu'**elle comprend :
a) un anticorps monoclonal ou polyclonal selon la revendication 25 ;
b) éventuellement des réactifs pour la constitution d'un milieu propice à la réaction immunologique ;
c) les réactifs permettant la détection du complexe antigène-anticorps produit lors de la réaction immunologique.

28. Procédé de détermination du caractère pluripotent d'une cellule ES d'oiseau, **caractérisé en ce qu'**on détermine la présence d'un produit d'expression du gène de séquence SEQ ID N°1 ou de l'ARNm de SEQ ID N°1.

29. Procédé selon la revendication 28, **caractérisé en ce que** la détection de l'ARNm de SEQ ID N°1 s'effectue par Northern Blot ou par RT-PCR en utilisant une sonde ou des amorces, par une utilisation selon la revendication 20.

30. Procédé selon la revendication 28, **caractérisé en ce que** l'on détecte la présence de la protéine SEQ ID N°2, par exemple en utilisant un anticorps selon la revendication 25.

31. Procédé de classification de l'appartenance d'un oiseau à l'ordre des Galliformes, **caractérisé en ce que** l'on détecte, dans le génome dudit oiseau, la présence d'une séquence d'acide nucléique choisie parmi un acide nucléique selon l'une des revendications 1 à 3, un fragment d'au moins 15 nucléotides consécutifs de la séquence SEQ ID N°1 et une séquence nucléique s'hybridant dans des conditions de forte stringence avec l'une de ces séquences.

32. Procédé de détermination de la présence d'un échantillon provenant d'un oiseau de l'ordre des Galliformes dans un échantillon alimentaire, **caractérisé en ce que** l'on détecte, dans ledit échantillon, la présence d'une séquence d'acide nucléique choisie parmi un acide nucléique selon l'une des revendications 1 à 3, un fragment d'au moins 15 nucléotides consécutifs de la séquence SEQ ID N°1 et une séquence nucléique s'hybridant dans des conditions de forte stringence avec l'une de ces séquences.

33. Puce à ADN **caractérisée en ce qu'**elle contient une séquence nucléique selon l'une des revendications 1 à 3.

34. Puce à protéines **caractérisée en ce qu'**elle contient un polypeptide selon l'une des revendications 4, 5 ou 24, ou un anticorps selon la revendication 25.

35. Procédé de détection et/ou de dosage d'une séquence d'acide nucléique choisie parmi un acide nucléique selon l'une des revendications 1 à 3 dans un échantillon biologique ou alimentaire, **caractérisé en ce qu'**il comprend les étapes suivantes :
a) mise en contact dudit échantillon avec une séquence d'acide nucléique marquée choisie parmi un polynucléotide selon l'une des revendications 1 à 3, un fragment d'au moins 15 nucléotides consécutifs de la séquence SEQ ID N°1 et une séquence nucléique s'hybridant dans des conditions de forte stringence avec l'une de ces séquences;
b) détection et/ou dosage de l'hybride formé entre ledit polynucléotide et l'acide nucléique dudit échantillon.

36. Procédé de détection et/ou de dosage d'une séquence d'acide nucléique choisie parmi un acide nucléique selon l'une des revendications 1 à 3 dans un échantillon biologique ou alimentaire, **caractérisé en ce qu'**il comprend une étape d'amplification des acides nucléiques dudit échantillon à l'aide d'amorces choisies parmi les acides nucléiques selon l'une des revendications 1 à 2, les fragments d'au moins 15 nucléotides consécutifs de la séquence SEQ ID N°1 et les séquences nucléiques s'hybridant dans des conditions de forte stringence avec l'une de ces séquences.

37. Procédé de criblage d'une substance ou d'un milieu capables d'induire une différentiation de cellules pluripotentes, **caractérisé en ce que** qu'il comprend les étapes suivantes :
a) maintien de cellules ES, à l'exception des cellules ES humaines, selon l'une des revendications 7 à 17 dans un milieu de culture permettant le maintien du phénotype pluripotent ;
b) ajout de ladite substance dans ledit milieu de culture ou remplacement dudit milieu de culture par le milieu à tester ;
c) détermination de l'induction de la différentiation par l'absence d'expression de la protéine SEQ ID N°2 ou du gène exogène.

38. Procédé selon la revendication 37, **caractérisée en ce qu'**il est effectué avec des cellules selon l'une des revendications 8 à 17.

39. Procédé selon la revendication 37 ou 38, **caractérisée en ce que** l'on utilise des cellules 9N2.5, et que l'on détecte l'absence d'expression de la β-galactosidase.

40. Procédé de criblage d'une substance capable de restaurer le caractère pluripotent de cellules différentiées dérivant de cellules selon l'une des revendications 8 à 17, **caractérisé en ce que** qu'il comprend les étapes suivantes :
a) maintien de cellules différentiées dérivant de cellules selon l'une des revendications 8 à 17 dans un milieu de culture adéquat ;
b) remplacement dudit milieu de culture par un milieu permettant de maintenir un phénotype pluripotent, et contenant ladite substance à tester ;
c) détermination de la restauration du caractère pluripotent desdites cellules par l'expression de la protéine SEQ ID N°2 ou du gène exogène, dans lesdites cellules.

41. Procédé selon la revendication 40, **caractérisé en ce que** l'on utilise des cellules 9N2.5 différentiées, et que l'on détecte l'expression de la β-galactosidase.

42. Composé **caractérisé en ce qu'**il est choisi parmi
a) un acide nucléique selon l'une des revendications 1 à 3 ;
b) un polypeptide selon l'une des revendications 4, 5 ou 24 ;
c) un vecteur selon la revendication 6 ;
d) une cellule selon l'une des revendications 7 à 17 ;
e) un anticorps selon la revendication 25,
à titre de médicament.

43. Utilisation de la séquence nucléotidique 3111-3670 de SEQ ID N°1 en tant que promoteur d'un gène d'intérêt pour une expression spécifique dudit gène d'intérêt dans des cellules pluripotentes aviaires.

## Claims

1. Purified or isolated nucleic acid **characterized in that** it comprises a nucleic acid sequence chosen from the group of following sequences:
a) SEQ ID No. 1, or the nucleotide fragment 1409-2878 of SEQ ID No. 1;
b) the nucleotide fragment 3111-3670 of SEQ ID No. 1;
c) a nucleic acid sequence having a percentage identity of at least 80%, after optimal alignment, with a sequence defined in a) or b), which has a role in the pluripotent nature of ES cells and which can be used as a marker for the pluripotent nature of ES cells;
d) the complementary sequence or the RNA sequence derived from the sequence as defined in a), b) or c).

2. Purified or isolated nucleic acid according to Claim 1, **characterized in that** it comprises or consists of SEQ ID No. 1, the complementary sequence or the RNA sequence derived from one of these sequences.

3. Purified or isolated nucleic acid, **characterized in that** it encodes a polypeptide which has a continuous fragment of at least 200 amino acids of the protein SEQ ID No. 2.

4. Isolated polypeptide, **characterized in that** it comprises a polypeptide chosen from:
a) a polypeptide of sequence SEQ ID No. 2;
b) a polypeptide comprising at least 80% identity with said polypeptide of a), which has a role in the pluripotent nature of ES cells and which can be used as a marker for the pluripotent nature of ES cells.

5. Polypeptide according to Claim 4, **characterized in that** it consists of a sequence chosen from SEQ ID No. 2, or a sequence having at least 80% identity with this sequence after optimal alignment.

6. Cloning and/or expression vector comprising a nucleic acid according to one of Claims 1 to 3 or encoding a polypeptide according to either of Claims 4 and 5.

7. Host cell, with the exception of human ES cells, **characterized in that** it is transformed with a vector according to Claim 6.

8. Host cell containing a nucleic acid according to one of Claims 1 to 3, **characterized in that** it is a bird ES cell also modified by introducing an exogenous gene, said exogenous gene being integrated into said nucleic acid according to one of Claims 1 to 3 and being expressed only and specifically when said cell is maintained in the pluripotent state.

9. Cell according to Claim 8, **characterized in that** said exogenous gene is a reporter gene.

10. Cell according to Claim 9, **characterized in that** said reporter gene is chosen from lacZ, GFP, luciferase, ROSA-β-geo and a gene for resistance to an antibiotic.

11. Host cell containing a nucleic acid according to one of Claims 1 to 3, **characterized in that** it is a bird cell also modified by introducing an exogenous nucleic acid, said exogenous nucleic acid being integrated into said nucleic acid according to one of Claims 1 to 3.

12. Cell according to Claim 11, **characterized in that** said exogenous nucleic acid is a gene of therapeutic interest, optionally preceded by a spatio-temporal promoter and/or by terminator sequences.

13. Cell according to Claim 11, **characterized in that** said exogenous nucleic acid is a genetic marker.

14. Cell according to one of Claims 8 to 13, **characterized in that** said bird belongs to the order Galliformes.

15. Cell according to Claim 14, **characterized in that** said bird is a chicken or a quail.

16. Cell according to either of Claims 14 and 15, **characterized in that** said reporter gene is integrated under the control of the promoter of the *ens-1* gene of nucleotide sequence 3111-3670 of SEQ ID No. 1.

17. Cell according to either of Claims 14 and 15, **characterized in that** it is a 9N2.5 cell, deposited with the Collection Nationale de Cultures de Microorganismes on May 11, 2000, under the identification number I-2477.

18. Differentiated bird cell, **characterized in that** it derives from an ES cell according to one of Claims 8 to 17.

19. Animal, except for human, **characterized in that** it comprises a cell according to one of Claims 7 to 18.

20. Use of a nucleic acid sequence chosen from a nucleic acid sequence according to one of Claims 1 to 3, a fragment of at least 15 consecutive nucleotides of the sequence SEQ ID No. 1 and a nucleic acid sequence which hybridizes with one of these sequences under high stringency conditions, as a probe or primer, for detecting and/or amplifying nucleic acid sequences according to one of Claims 1 to 3.

21. Use of a nucleic acid sequence chosen from a nucleic acid according to one of Claims 1 to 3, a fragment of at least 15 consecutive nucleotides of the sequence SEQ ID No. 1 and a nucleic acid sequence which hybridizes with one of these sequences under high stringency conditions, as a sense or antisense oligonucleotide specific for a nucleic acid sequence according to one of Claims 1 to 3.

22. Use of a nucleic acid sequence according to one of Claims 1 to 3, for producing a recombinant polypeptide according to Claim 4 or 5.

23. Method for obtaining a recombinant polypeptide, **characterized in that** a cell according to Claim 7 is cultured under conditions which allow the expression of said polypeptide, and **in that** said recombinant polypeptide is recovered, said polypeptide being the polypeptide according to Claim 4 or 5.

24. Recombinant polypeptide, **characterized in that** it is obtained using a method according to Claim 23.

25. Monoclonal or polyclonal antibody, **characterized in that** it selectively binds a polypeptide according to one of Claims 4, 5 and 24.

26. Method for detecting a polypeptide according to one of Claims 4, 5 and 24, **characterized in that** it comprises the following steps:
a) bringing a biological sample into contact with an antibody according to Claim 25;
b) demonstrating the antigen-antibody complex formed.

27. Kit of reagents for carrying out a method according to Claim 26, **characterized in that** it comprises:
a) a monoclonal or polyclonal antibody according to Claim 25;
b) optionally, reagents for constituting a medium suitable for the immunoreaction;
c) the reagents for detecting the antigen-antibody complex produced during the immunoreaction.

28. Method for determining the pluripotent nature of a bird ES cell, **characterized in that** the presence of a product of expression of the gene of sequence SEQ ID No. 1, or of the mRNA of SEQ ID No. 1, is determined.

29. Method according to Claim 28, **characterized in that** the mRNA of SEQ ID No. 1 is detected by Northern blotting or by RT-PCR using a probe or primers, by the use according to Claim 20.

30. Method according to Claim 28, **characterized in that** the presence of the protein SEQ ID No. 2 is detected, for example using an antibody according to Claim 25.

31. Method for classifying a bird as belonging to the order Galliformes, **characterized in that** the presence of a nucleic acid sequence chosen from a nucleic acid according to one of Claims 1 to 3, a fragment of at least 15 consecutive nucleotides of the sequence SEQ ID No. 1 and a nucleic acid sequence which hybridizes with one of these sequences under high stringency conditions, is detected in the genome of said bird.

32. Method for determining the presence of a sample originating from a bird of the order Galliformes in a food sample, **characterized in that** the presence of a nucleic acid sequence chosen from a nucleic acid according to one of Claims 1 to 3, a fragment of at least 15 consecutive nucleotides of the sequence SEQ ID No. 1 and a nucleic acid sequence which hybridizes with one of these sequences under high stringency conditions, is detected in said sample.

33. DNA chip, **characterized in that** it contains a nucleic acid sequence according to one of Claims 1 to 3.

34. Protein chip, **characterized in that** it contains a polypeptide according to one of Claims 4, 5 and 24, or an antibody according to Claim 25.

35. Method for detecting and/or assaying a nucleic acid sequence chosen from a nucleic acid according to one of Claims 1 to 3, in a biological or food sample, **characterized in that** it comprises the following steps:
a) bringing said sample into contact with a labelled nucleic acid sequence chosen from a polynucleotide according to one of Claims 1 to 3, a fragment of at least 15 consecutive nucleotides of the sequence SEQ ID No. 1 and a nucleic acid sequence which hybridizes with one of these sequences under high stringency conditions;
b) detecting and/or assaying the hybrid formed between said polynucleotide and the nucleic acid of said sample.

36. Method for detecting and/or assaying a nucleic acid sequence chosen from a nucleic acid according to one of Claims 1 to 3, in a biological or food sample, **characterized in that** it comprises a step of amplification of the nucleic acids of said sample using primers chosen from the nucleic acids according to either of Claims 1 and 2, the fragments of at least 15 consecutive nucleotides of the sequence SEQ ID No. 1 and the nucleic acid sequences which hybridize with one of these sequences under high stringency conditions.

37. Method for screening for a substance or for a medium capable of inducing differentiation of pluripotent cells, **characterized in that** it comprises the following steps:
a) maintaining ES cells, with the exception of human ES cells, according to one of Claims 7 to 17 in a culture medium making it possible to maintain the pluripotent phenotype;
b) adding said substance to said culture medium or replacing said culture medium with the medium to be tested;
c) determining the induction of differentiation by the absence of expression of the protein SEQ ID No. 2 or of the exogenous gene.

38. Method according to Claim 37, **characterized in that** it is carried out with cells according to one of Claims 8 to 17.

39. Method according to Claim 37 or 38, **characterized in that** 9N2.5 cells are used, and **in that** the absence of expression of β-galactosidase is detected.

40. Method for screening for a substance capable of restoring the pluripotent nature of differentiated cells derived from cells according to one of Claims 8 to 17, **characterized in that** it comprises the following steps:
a) maintaining differentiated cells derived from cells according to one of Claims 8 to 17 in a suitable culture medium;
b) replacing said culture medium with a medium which makes it possible to maintain a pluripotent phenotype and which contains said substance to be tested;
c) determining the restoration of the pluripotent nature of said cells by the expression of the protein SEQ ID No. 2 or of the exogenous gene, in said cells.

41. Method according to Claim 40, **characterized in that** differentiated 9N2.5 cells are used, and **in that** the expression of β-galactosidase is detected.

42. Compound, **characterized in that** it is chosen from
a) a nucleic acid according to one of Claims 1 to 3;
b) a polypeptide according to one of Claims 4, 5 or 24;
c) a vector according to Claim 6;
d) a cell according to one of Claims 7 to 17;
e) an antibody according to Claim 25;
as a medicinal product.

43. Use of the nucleotide sequence 3111-3670 of SEQ ID No. 1, as a promoter of a gene of interest, for specific expression of said gene of interest in avian pluripotent cells.

## Patentansprüche

1. Gereinigte oder isolierte Nukleinsäure, **dadurch gekennzeichnet, dass** sie eine Nukleinsequenz umfasst, die aus der Gruppe der folgenden Sequenzen ausgewählt ist:
a) der SEQ ID Nr. 1 oder dem Nukleotidfragment 1409-2878 der SEQ ID Nr. 1,
b) dem Nukleotidfragment 3111-3670 der SEQ ID Nr. 1,
c) einer Nukleinsequenz, die nach optimaler Ausrichtung mit einer in a) oder b) definierten Sequenz mindestens 80 % Identität aufweist, eine Rolle beim pluripotenten Charakter der ES Zellen besitzt und als Marker des pluripotenten Charakters der ES Zellen verwendbar ist,
d) der komplementären Sequenz oder der definierten Sequenz abgeleiteten RNA-Sequenz aus der wie in a), b) oder c).

2. Gereinigte oder isolierte Nukleinsäure nach Anspruch 1, **dadurch gekennzeichnet, dass** sie SEQ ID Nr. 1 umfasst oder von ihr gebildet wird, die komplementäre Sequenz oder die aus einer dieser Sequenzen abgeleiteten RNA-Sequenz.

3. Gereinigte oder isolierte Nukleinsäure, **dadurch gekennzeichnet, dass** sie für ein Polypeptid kodiert, das ein ununterbrochenes Fragment von mindestens 200 Aminosäuren des Proteins SEQ ID Nr. 2 besitzt.

4. Isoliertes Polypeptid, **dadurch gekennzeichnet, dass** es ein Polypeptid umfasst, das ausgewählt ist aus:
a) einem Polypeptid der Sequenz SEQ ID Nr. 2,
b) einem Polypeptid, das mit dem Polypeptid von a) mindestens 80 % Identität aufweist, eine Rolle beim pluripotenten Charakter der ES Zellen besitzt und als Marker des pluripotenten Charakters der ES Zellen verwendbar ist.

5. Polypeptid nach Anspruch 4, **dadurch gekennzeichnet, dass** es von einer Sequenz gebildet wird, die aus SEQ ID Nr. 2 ausgewählt ist oder einer Sequenz, die nach optimaler Ausrichtung mindestens 80 % Identität mit dieser Sequenz besitzt.

6. Klonierungs- und/oder Expressionsvektor, der eine Nukleinsäure nach einem der Ansprüche 1 bis 3 umfasst oder für ein Polypeptid nach einem der Ansprüche 4 und 5 kodiert.

7. Wirtszelle mit Ausnahme der humanen ES Zellen, **dadurch gekennzeichnet, dass** sie von einem Vektor nach Anspruch 6 transformiert ist.

8. Wirtszelle, die eine Nukleinsäure nach einem der Ansprüche 1 bis 3 enthält, **dadurch gekennzeichnet, dass** es sich um eine embryonale ES Vogelzelle handelt, die weiterhin durch Einführung eines exogenen Gens modifiziert wurde, wobei das exogene Gen in die Nukleinsäure nach einem der Ansprüche 1 bis 3 integriert ist und nur dann und spezifisch exprimiert wird, wenn die Zelle im pluripotenten Zustand erhalten wird.

9. Zelle nach Anspruch 8, **dadurch gekennzeichnet, dass** das exogene Gen ein Reportergen ist.

10. Zelle nach Anspruch 9, **dadurch gekennzeichnet, dass** das Reportergen aus LacZ, GFP, Luciferase, ROSA-β-geo, einem Resistenzgen gegenüber einen Antibiotikum ausgewählt ist.

11. Wirtszelle, die eine Nukleinsäure nach einem der Ansprüche 1 bis 3 enthält, **dadurch gekennzeichnet, dass** es sich um eine im Weiteren durch Einführen einer exogenen Nukleinsäure modifizierte Vogelzelle handelt, wobei die exogene Nukleinsäure in die Nukleinsäure nach einem der Ansprüche 1 bis 3 integriert ist.

12. Zelle nach Anspruch 11, **dadurch gekennzeichnet, dass** die exogene Nukleinsäure ein therapeutisch bedeutsames Gen ist, dem eventuell ein Raum-Zeit-Promoter und/oder terminierende Sequenzen vorangehen.

13. Zelle nach Anspruch 11, **dadurch gekennzeichnet, dass** die exogene Nukleinsäure ein genetisch Marker ist.

14. Zelle nach einem der Ansprüche 8 bis 13, **dadurch gekennzeichnet, dass** der Vogel der Ordnung der Hühnervögel angehört.

15. Zelle nach Anspruch 14, **dadurch gekennzeichnet, dass** der Vogel ein Huhn oder eine Wachtel ist.

16. Zelle nach einem der Ansprüche 14 und 15, **dadurch gekennzeichnet, dass** das Reportergen unter der Kontrolle des Promoters des Gens *ens-*1 der Nukleotidsequenz 3111-3670 der SEQ ID Nr. 1 integriert wird.

17. Zelle nach einem der Ansprüche 14 und 15, **dadurch gekennzeichnet, dass** es sich um eine Zelle 9N2.5 handelt, die bei der Collection Nationale des Microorganismes am 11. Mai 2000 unter der laufenden Nummer I-2477 hinterlegt wurde.

18. Differenzierte Vogelzelle, **dadurch gekennzeichnet, dass** sie aus einer ES Zelle nach einem der Ansprüche 8 bis 17 abgeleitet ist.

19. Tier mit Ausnahme des Menschen, **dadurch gekennzeichnet, dass** es eine Zelle nach einem der Ansprüche 7 bis 18 umfasst.

20. Verwendung einer Nukleinsäuresequenz, die aus einer Nukleinsäuresequenz nach einem der Ansprüche 1 bis 3 ausgewählt ist, einem Fragment von mindestens 15 aufeinanderfolgenden Nukleotiden der Sequenz SEQ ID Nr. 1 und einer Nukleinsequenz, die unter Bedingungen starker Stringenz mit einer dieser Sequenzen hybridisiert, als Sonde oder Primer für die Detektion und/oder Amplifikation von Nukleinsäuren nach einem der Ansprüche 1 bis 3.

21. Verwendung einer Nukleinsäuresequenz, die aus einer Nukleinsäure nach einem der Ansprüche 1 bis 3 ausgewählt ist, einem Fragment von mindestens 15 aufeinanderfolgenden Nukleotiden der Sequenz SEQ ID Nr. 1 und einer Nukleinsequenz, die unter Bedingungen starker Stringenz mit einer dieser Sequenzen hybridisiert, als spezifisches Sense- oder Antisense-Oligonukleotid einer Nukleinsäuresequenz nach einem der Ansprüche 1 bis 3.

22. Verwendung einer Nukleinsäuresequenz nach einem der Ansprüche 1 bis 3 zur Herstellung eines rekombinanten Polypeptids nach Anspruch 4 oder 5.

23. Verfahren zur Herstellung eines rekombinanten Polypeptids, **dadurch gekennzeichnet, dass** man eine Zelle nach Anspruch 7 unter Bedingungen kultiviert, die die Expression des Polypeptids erlauben und dass man das rekombinante Polypeptid gewinnt, wobei das Polypeptid das Polypeptid nach Anspruch 4 oder 5 ist.

24. Rekombinantes Polypeptid, **dadurch gekennzeichnet, dass** es durch ein Verfahren nach Anspruch 23 hergestellt wird.

25. Monoklonaler oder polyklonaler Antikörper, **dadurch gekennzeichnet, dass** er selektiv ein Polypeptid nach einem der Ansprüche 4, 5 oder 24 bindet.

26. Verfahren zur Detektion eines Polypeptids nach einem der Ansprüche 4, 5 oder 24, **dadurch gekennzeichnet, dass** es die folgenden Schritte umfasst:
a) Herstellung des Kontakts einer biologischen Probe mit einem Antikörper nach Anspruch 25,
b) Feststellung des gebildeten Antigen-Antikörper-Komplexes.

27. Reagenzkit für die Durchführung eines Verfahrens nach Anspruch 26, **dadurch gekennzeichnet, dass** es umfasst:
a) einen monoklonalen oder polyklonalen Antikörper nach Anspruch 25,
b) eventuell Reagenzien zur Herstellung eines für die immunologische Reaktion günstigen Mediums,
c) Reagenzien, die die Detektion des bei der immunologischen Reaktion hergestellten Antigen-Antikörper-Komplexes erlauben.

28. Verfahren zur Bestimmung des pluripotenten Charakters einer ES Zelle, **dadurch gekennzeichnet, dass** das Vorhandensein eines Expressionsprodukts des Gens der Sequenz SEQ ID Nr. 1 oder der mRNA der Sequenz ID Nr. 1 bestimmt wird.

29. Verfahren nach Anspruch 28, **dadurch gekennzeichnet, dass** die Detektion der mRNA der SEQ ID Nr. 1 durch Northern Blot oder durch RT-PCR bei Verwendung einer Sonde oder von Primern erfolgt, durch eine Verwendung nach Anspruch 20.

30. Verfahren nach Anspruch 28, **dadurch gekennzeichnet, dass** man das Vorhandensein des Proteins SEQ ID Nr. 2 ermittelt, zum Beispiel durch Verwendung eines Antikörpers nach Anspruch 25.

31. Verfahren zur Klassifizierung der Zugehörigkeit eines Vogels zur Ordnung der Hühnervögel, **dadurch gekennzeichnet, dass** man im Genom des Vogels das Vorhandensein einer Nukleinsäuresequenz ermittelt, die aus einer Nukleinsäure nach einem der Ansprüche 1 bis 3, einem Fragment von mindestens 15 aufeinanderfolgenden Nukleotiden der Sequenz SEQ ID Nr. 1 und einer Nukleinsequenz, die unter Bedingungen starker Stringenz mit einer dieser Sequenzen hybridisiert, ausgewählt ist.

32. Verfahren zur Ermittlung des Vorhandenseins einer Probe eines Vogels aus der Ordnung der Hühnervögel in einer Lebensmittelprobe, **dadurch gekennzeichnet, dass** man in der Probe das Vorhandensein einer Nukleinsäuresequenz ermittelt, die aus einer Nukleinsäure nach einem der Ansprüche 1 bis 3, einem Fragment von mindestens 15 aufeinanderfolgenden Nukleotiden der Sequenz SEQ ID Nr. 1 und einer Nukleinsequenz, die unter Bedingungen starker Stringenz mit einer dieser Sequenzen hybridisiert, ausgewählt ist.

33. DNA-Chip, **dadurch gekennzeichnet, dass** er eine Nukleotidsequenz nach einem der Ansprüche 1 bis 3 enthält.

34. Proteinchip, **dadurch gekennzeichnet, dass** er ein Polypeptid nach einem der Ansprüche 4, 5 oder 24 oder einen Antikörper nach Anspruch 25 enthält.

35. Verfahren zur Detektion und/oder Bestimmung einer Nukleinsäuresequenz, die aus einer Nukleinsäure nach einem der Ansprüche 1 bis 3 in einer biologischen oder Lebensmittelprobe ausgewählt ist, **dadurch gekennzeichnet, dass** es die folgenden Schritte umfasst:
a) Herstellung des Kontakts der Probe mit einer markierten Nukleinsäuresequenz, die aus einem Polynukleotid nach einem der Ansprüche 1 bis 3, einem Fragment von mindestens 15 aufeinanderfolgenden Nukleotiden der Sequenz SEQ ID Nr. 1 und einer Nukleinsequenz, die unter Bedingungen starker Stringenz mit einer dieser Sequenzen hybridisiert, ausgewählt ist,
b) Detektion und/oder Bestimmung des zwischen dem Polynukleotid und der Nukleinsäure der Probe gebildeten Hybriden.

36. Verfahren zur Detektion und/oder Bestimmung einer Nukleinsäuresequenz, die aus einer Nukleinsäure nach einem der Ansprüche 1 bis 3 aus einer biologischen oder Lebensmittelprobe ausgewählt ist, **dadurch gekennzeichnet, dass** es einen Schritt der Amplifizierung der Nukleinsäuren der Probe mit Hilfe von Primern umfasst, die aus den Nukleinsäuren nach einem der Ansprüche 1 bis 2, den Fragmenten von mindestens 15 aufeinanderfolgenden Nukleotiden der Sequenz SEQ ID Nr. 1 und den Nukleinsequenzen, die unter Bedingungen starker Stringenz mit einer dieser Sequenzen hybridisieren, ausgewählt sind.

37. Screeningverfahren einer Substanz oder eines Mediums, die imstande sind, eine Differenzierung pluripotenter Zellen einzuleiten, **dadurch gekennzeichnet, dass** es die folgenden Schritte umfasst:
a) Aufrechterhalten ES Zellen, mit Ausnahme humaner ES Zellen, nach einem der Ansprüche 7 bis 17 in einem Kulturmedium, das die Aufrechterhaltung des pluripotenten Phänotyps erlaubt,
b) Hinzufügen der Substanz in das Kulturmedium oder Ersetzen des Kulturmediums durch das zu testende Medium,
c) Bestimmen der Induktion der Differenzierung durch Abwesenheit von Expression des Proteins SEQ ID Nr. 2 oder des exogenen Gens.

38. Verfahren nach Anspruch 37, **dadurch gekennzeichnet, dass** es mit Zellen nach einem der Ansprüche 8 bis 17 durchgeführt wird.

39. Verfahren nach Anspruch 37 oder 38, **dadurch gekennzeichnet, dass** 9N2.5-Zellen verwendet werden und dass die Abwesenheit von Expression der β-Galactosidase festgestellt wird.

40. Screeningverfahren einer Substanz, die imstande ist, den pluripotenten Charakter differenzierter Zellen, die von Zellen nach einem der Ansprüche 8 bis 17 abgeleitet sind, wiederherzustellen, **dadurch gekennzeichnet, dass** es die folgenden Schritte umfasst:
a) Aufrechterhalten differenzierter Zellen, die von Zellen nach einem der Ansprüche 8 bis 17 abgeleitet sind, in einem adäquaten Kulturmedium,
b) Ersetzen des Kulturmediums durch ein Medium, das es erlaubt, einen pluripotenten Phänotyp aufrechtzuerhalten, und das die zu testende Substanz enthält,
c) Bestimmen der Wiederherstellung des pluripotenten Charakters der Zellen durch Expression des Proteins SEQ ID Nr. 2 oder des exogenen Gens in den Zellen.

41. Verfahren nach Anspruch 40, **dadurch gekennzeichnet, dass** differenzierte 9N2.5-Zellen verwendet werden und dass die Expression der β-Galactosidase festgestellt wird.

42. Verbindung, **dadurch gekennzeichnet, dass** sie ausgewählt ist aus
a) einer Nukleinsäure nach einem der Ansprüche 1 bis 3,
b) einem Polypeptid nach einem der Ansprüche 4, 5 oder 24,
c) einem Vektor nach Anspruch 6,
d) einer Zelle nach einem der Ansprüche 7 bis 17,
e) einem Antikörper nach Anspruch 25 als Arzneimittel.

43. Verwendung der Nukleotidsequenz 3111-3670 von SEQ ID Nr. 1 als Promoter eines bedeutsamen Gens für eine spezifische Expression des bedeutsamen Gens in pluripotenten Vogelzellen.
